# EUROPEAN PATENT APPLICATION

(11) **EP 3 936 869 A2**
(43) Date of publication of application: **12.01.2022**
(21) Application number: 20766012.7
(22) Date of filing: 06.03.2020
(51) Int. Cl.: G01N 33/68, C12Q 1/6883

(54) **COMPOSITE MARKER FOR DIAGNOSIS OF AGE RELATED MACULAR DEGENERATION AND USE THEREOF**

(30) Priority: 07.03.2019 KR 20190026303
(71) Applicant: Reti Mark Co., Ltd., Seoul 04385 (KR)
(72) Inventor: PARK, Seong Jun, Seoul 02799 (KR); LEE, Young Ju, Suwon-si, Gyeonggi-do 16225 (KR); KIM, Hye Rim, Incheon 22792 (KR)
(74) Representative: Treeby, Philip David William
(86) International application number: PCT/KR2020/003169
(87) International publication number: WO 2020/180147

(57) **Abstract**

The present invention relates to a composite marker for diagnosis of age related macular degeneration (AMD) and a use of same and, more specifically, to a composite markers for diagnosis of age related macular degeneration, which consists of two or more blood markers specific to diagnosis of age related macular degeneration and has improved diagnostic performance. In addition, the present invention relates to a composition for diagnosis of age related macular degeneration, a diagnostic kit, and a method for providing information necessary for diagnosing age related macular degeneration, each of which uses the composite marker. The composite marker for diagnosis of age related macular degeneration of the present invention was found to be superior to combinations of other markers in terms of sensitivity and diagnosis performance and to exhibit a high diagnostic potential for both initial and late macular degeneration, as analyzed for combinations between protein quantitation values of the composite marker and fundamental clinical information. In addition, the composite marker of the present invention does not use blood proteins for biopsy, but can conveniently perform analysis using patient plasma and as such, can be advantageously applied to the early diagnosis of age related macular degeneration.

## Description

### CROSS-REFERENCE TO RELATED APPLICATION

This application claims priority to and the benefit of Korean Patent Application No. 10-2019-0026303, filed on 07.03.2019, the disclosure of which is incorporated herein by reference in its entirety.

### [Technical Field]

The present invention relates to a combined biomarker for diagnosing age-related macular degeneration (AMD) and a use thereof, and more specifically to a combined biomarker for diagnosing age-related macular degeneration which is composed of two or more blood biomarkers specific for the diagnosis of age-related macular degeneration with increased diagnostic performance. In addition, the present invention relates to a composition for diagnosing age-related macular degeneration, a diagnostic kit and a method for providing information necessary for the diagnosis of age-related macular degeneration, using the combined biomarker.

### [Background Art]

Age-related macular degeneration (AMD) is one of the three ophthalmic blindness diseases along with diabetic retinopathy and glaucoma as a disease resulting from various changes in the macula, which is the central part of the retina, and it mainly occurs in adults over 50 years of age. In developed countries, it is the most leading cause of adult blindness, and the incidence rate increases with increasing age. When the prevalence of age-related macular degeneration in Korea is considered based on the National Health and Nutrition Examination Survey, 11.7% of the population aged 60 to 69 and 18% of the population aged 70 and over are affected by age-related macular degeneration among Koreans. The macula refers to the central region of a neural tissue called the retina, and photoreceptor cells that respond to light stimulation are concentrated and responsible for central vision. A disease in which macular photoreceptor cells are lost with age and cause vision impairment is referred to as age-related macular degeneration. AMD is a degenerative disease affecting the retina, the retinitis pigmentosa (RPE), the Bruch's membrane, and the choroid.

AMD can be divided into a dry or non-exudative form and a wet or exudative form. The non-exudative form refers to a case where a lesion such as drusen or atrophy of the retinal pigment epithelium occurs in the retina. It usually does not cause severe blindness but can develop into a wet form. The exudative form is a case where choroidal neovasculatures are growing beneath the retina. These neovasculatures cause exudates, bleeding and the like in the macula, damaging photoreceptors and thus lowering central vision, mostly resulting in legal blindness of 0.1 or less when not treated. Macular degeneration in the exudative form has a very rapid rate of progression, often resulting in rapid deterioration of vision within a few weeks. Early detection of age-related macular degeneration is generally very important as it often cannot restore previous vision once vision impairment begins. Early detection is possible through regular ophthalmological examination by ophthalmologists, and if age-related macular degeneration is suspected by ophthalmic examination including fundus examination, detailed ophthalmic examinations such as fluorescein angiography, optical coherence tomography and the like can be performed to confirm the diagnosis.

Accordingly, as a result of attempting to develop a blood protein biomarker having high sensitivity and specificity and using the same in combination to enhance the early diagnostic capacity of age-related macular degeneration, the inventors of the present invention have confirmed that the combined biomarker including insulin-like growth factor binding protein 2 (IGFBP2), ADAMTS-like protein 2 (ADAMTSL2), galectin 3 binding protein (LGALS3BP) and complement factor H (CFH) has excellent diagnostic efficiency for age-related macular degeneration, and thereby completed the present invention.

### [Disclosure]

### [Technical Problem]

An object of the present invention is to provide a combined biomarker for diagnosing age-related macular degeneration, including blood biomarkers specific for the diagnosis of age-related macular degeneration.

Another object of the present invention is to provide a composition or a diagnostic kit for diagnosing age-related macular degeneration using the combined biomarker for diagnosing age-related macular degeneration.

Still another object of the present invention is to provide a method for providing information necessary for the diagnosis of age-related macular degeneration using the combined biomarker for diagnosing age-related macular degeneration.

### [Technical Solution]

In order to achieve the above objects, the present invention provides a combined biomarker for diagnosing age-related macular degeneration, including insulin-like growth factor binding protein 2 (IGFBP2), ADAMTS-like protein 2 (ADAMTSL2), galectin 3 binding protein (LGALS3BP) and complement factor H (CFH).

In a preferred exemplary embodiment of the present invention, the combined biomarker may further include one or more biomarkers selected from the group consisting of ceruloplasmin (Cp), protein DDI1 homolog 2 (DDI2), ficolin 2 (FCN2), mannose-binding protein C (MBL2), pancreatic triacylglycerol lipase (PNLIP), E-selectin (SELE), sialic acid-binding Ig-like lectin 14 (SIGLEC14), thrombospondin-1 (THBS1) and zymogen granule protein 16 homolog B (ZG16B).

In addition, the present invention provides a composition for diagnosing age-related macular degeneration, including an agent for measuring the mRNA or protein level of a combined biomarker for diagnosing age-related macular degeneration, which includes insulin-like growth factor binding protein 2 (IGFBP2), ADAMTS-like protein 2 (ADAMTSL2), galectin 3 binding protein (LGALS3BP) and complement factor H (CFH).

In a preferred exemplary embodiment of the present invention, the combined biomarker may further include one or more biomarkers selected from the group consisting of ceruloplasmin (Cp), protein DDI1 homolog 2 (DDI2), ficolin 2 (FCN2), mannose-binding protein C (MBL2), pancreatic triacylglycerol lipase (PNLIP), E-selectin (SELE), sialic acid-binding Ig-like lectin 14 (SIGLEC14), thrombospondin-1 (THBS1) and zymogen granule protein 16 homolog B (ZG16B).

In another preferred exemplary embodiment of the present invention, the agent for measuring the mRNA level of the combined biomarker may be a primer pair, probe or antisense nucleotide that specifically binds to a gene of each biomarker.

In still another preferred exemplary embodiment of the present invention, the agent for measuring the protein level of the combined biomarker may include an antibody, interacting protein, ligand, nanoparticle or aptamer that specifically binds to a protein or peptide fragment of each biomarker.

In addition, the present invention provides a kit for diagnosing age-related macular degeneration, including the composition for diagnosing age-related macular degeneration.

In a preferred exemplary embodiment of the present invention, the kit may be a reverse transcription polymerase chain reaction (RT-PCR) kit, a DNA chip kit, an enzyme-linked immunosorbent assay (ELISA) kit, a protein chip kit, a rapid kit or a multiple reaction monitoring (MRM) kit.

In addition, the present invention provides a method for providing information for diagnosing age-related macular degeneration, including (a) measuring the mRNA or protein level of the combined biomarker for diagnosing age-related macular degeneration, including insulin-like growth factor binding protein 2 (IGFBP2), ADAMTS-like protein 2 (ADAMTSL2), galectin 3 binding protein (LGALS3BP) and complement factor H (CFH), from a biological sample of a patient; and (b) comparing the mRNA or protein expression level with an mRNA or protein expression level from a sample of a control group.

In a preferred exemplary embodiment of the present invention, the combined biomarker may further include one or more biomarkers selected from the group consisting of ceruloplasmin (Cp), protein DDI1 homolog 2 (DDI2), ficolin 2 (FCN2), mannose-binding protein C (MBL2), pancreatic triacylglycerol lipase (PNLIP), E-selectin (SELE), sialic acid-binding Ig-like lectin 14 (SIGLEC14), thrombospondin-1 (THBS1) and zymogen granule protein 16 homolog B (ZG16B).

In another preferred exemplary embodiment of the present invention, the method for providing information for diagnosing age-related macular degeneration compares with the control group by further including one or more types of clinical information selected from the group consisting of the patient's age, body mass index (BMI), hypertension, hyperlipidemia, smoking status and cardiovascular disease.

In still another exemplary embodiment of the present invention, the method for providing information for diagnosing age-related macular degeneration may further include diagnosing age-related macular degeneration if the gene expression level or protein expression level of the combined biomarker increases compared to the control group.

In still another exemplary embodiment of the present invention, the measurement of the mRNA expression level may be performed by using a reverse transcription polymerase chain reaction, a competitive reverse transcription polymerase chain reaction, a real-time reverse transcription polymerase chain reaction, an RNase protection assay, Northern blotting or a DNA chip.

In still another exemplary embodiment of the present invention, the measurement of the protein expression level may be performed by using multiple reaction monitoring (MRM), parallel reaction monitoring (PRM), sequential windowed data independent acquisition of the total high-resolution (SWATH), selected reaction monitoring (SRM) or immune-multiple reaction monitoring (iMRM).

In still another exemplary embodiment of the present invention, the analysis of step (b) may be performed by a statistical analysis method.

In still another exemplary embodiment of the present invention, the statistical analysis method may be selected from the group consisting of a linear or non-linear regression analysis method, a linear or non-linear classification analysis method, a logistic regression analysis method, an analysis of variance (ANOVA), a neural network analysis method, a genetic analysis method, a support vector machine analysis method, a hierarchical analysis or clustering analysis method, a hierarchical algorithm or kernel principal component analysis method using decision tree, the Markov Blanket analysis method, a recursive feature elimination or entropy-based regression feature elimination analysis method, a forward floating search or backward floating search analysis method, and a combination thereof.

### [Advantageous Effects]

The combined biomarker for diagnosing age-related macular degeneration according to the present invention was confirmed to exhibit excellent sensitivity and diagnostic performance compared to other biomarker combinations, and it was also confirmed to exhibit high diagnostic capacity in two stages of early and late age-related macular degeneration when the protein quantitative values of the combined biomarker and the basic clinical information were combined and analyzed. In addition, the combined biomarker of the present invention can be effectively used for the early diagnosis of age-related macular degeneration because it can be conveniently analyzed using the patient plasma without using a biopsy as a blood protein.

### [Description of Drawings]

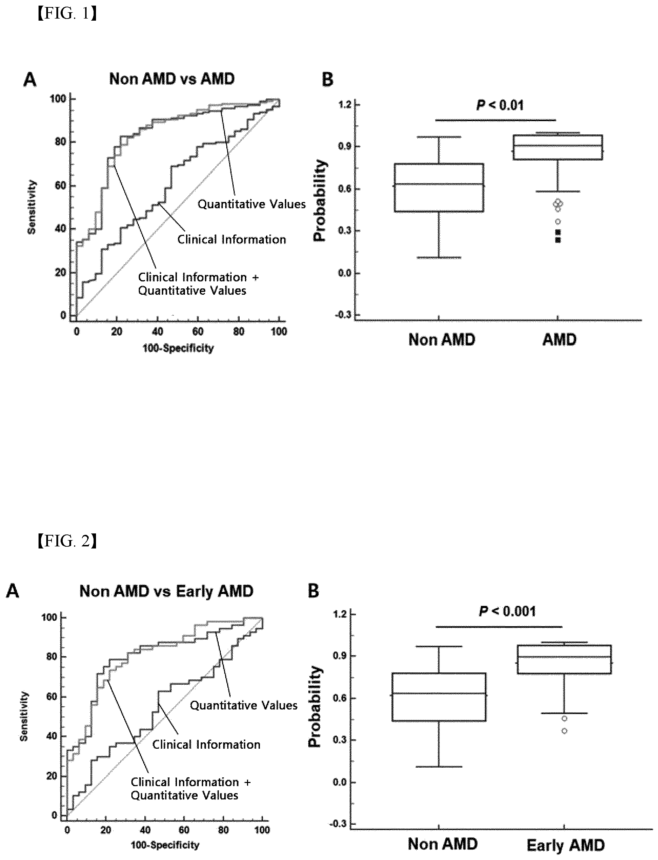

FIG. 1 is data obtained by combining the protein quantification values of a combined biomarker and the basic clinical information of all age-related macular degeneration patients, and analyzed by statistical processing with a logistic regression model (A) and T-test (B) (AMD: age-related macular degeneration, Non AMD: non-macular degeneration).
FIG. 2 is data obtained by combining the protein quantification values of a combined biomarker and the basic clinical information of patients with early age-related macular degeneration, and analyzed by statistical processing with a logistic regression model (A) and T-test (B) (early AMD: early age-related macular degeneration, Non AMD: non-age related macular degeneration).

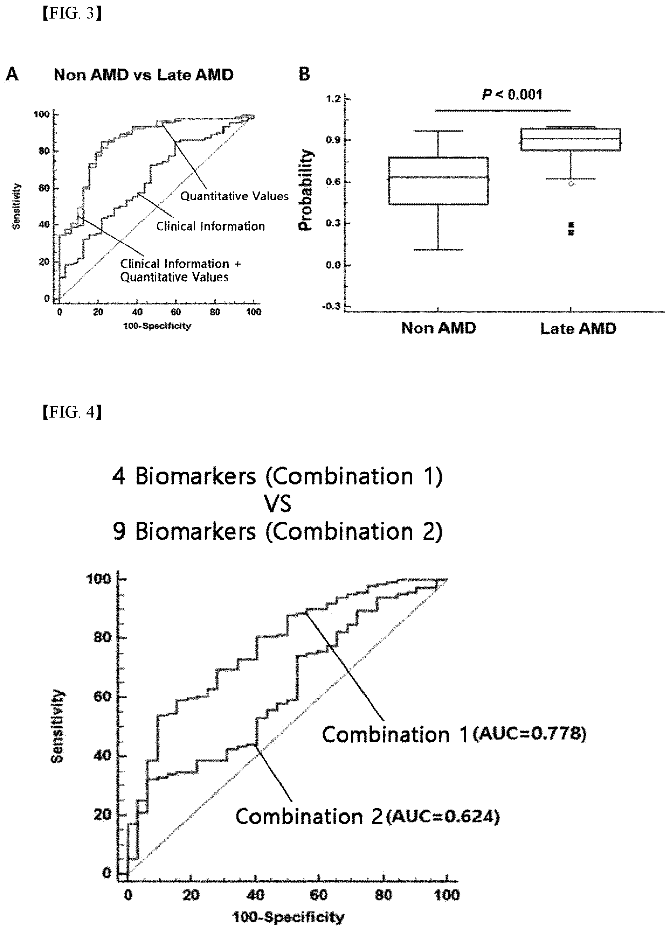

FIG. 3 is data obtained by combining the protein quantification values of a combined biomarker and the basic clinical information of patients with late age-related macular degeneration, and analyzed by statistical processing with a logistic regression model (A) and T-test (B) (late AMD: late age-related macular degeneration, Non AMD: non-age-related macular degeneration).
FIG. 4 is data obtained by combining the protein quantitative values according to a combination of IGFBP2, ADAMTSL2, LGALS3BP and CHF (Combination 1) and a combination of CP, DDI, FCN2, MBL2, SIGLEC14, SELE, PNLIP, THBS1 and ZG16B (Combination 2) among the 13 biomarkers identified in the present invention and the basic clinical information of patients, and analyzed by statistical processing with a logistic regression model.

### [Modes of the Invention]

Hereinafter, the present invention will be described in detail.

In one aspect, the present invention relates to a combined biomarker for diagnosing age-related macular degeneration, including insulin-like growth factor binding protein 2 (IGFBP2), ADAMTS-like protein 2 (ADAMTSL2), galectin 3 binding protein (LGALS3BP) and complement factor H (CFH).

As used herein, the term "diagnosis" means identifying the presence or characteristics of a pathological condition. For the purposes of the present invention, the diagnosis is to determine whether age-related macular degeneration has occurred.

As used herein, the term "biomarker for diagnosing" includes organic biomolecules such as polypeptides or nucleic acids (*e.g.,* mRNA, *etc*.), lipids, glycolipids, glycoproteins, sugars (monosaccharides, disaccharides, oligosaccharides, *etc*.) and the like, which show a significantly increased or decreased pattern of the gene expression level or protein expression level in a subject with age-related macular degeneration as compared to a normal control group (a subject with no age-related macular degeneration).

Depending on the degree of progression, age-related macular degeneration is classified into early age-related macular degeneration (early AMD) and late age-related macular degeneration (late AMD). Early age-related macular degeneration is characterized by no vascular development, and since late age-related macular degeneration differs in the mechanism such as the development of blood vessels, even a biomarker known as a biomarker for the diagnosis of late age-related macular degeneration may not necessarily be used as a biomarker for the diagnosis of early age-related macular degeneration.

The combined biomarker of the present invention may specifically diagnose both early age-related macular degeneration and late age-related macular degeneration.

In the present invention, the combined biomarker may further include one or more biomarkers selected from the group consisting of ceruloplasmin (Cp), protein DDI1 homolog 2 (DDI2), ficolin 2 (FCN2), mannose-binding protein C (MBL2), pancreatic triacylglycerol lipase (PNLIP), E-selectin (SELE), sialic acid-binding Ig-like lectin 14 (SIGLEC14), thrombospondin-1 (THBS1) and zymogen granule protein 16 homolog B (ZG16B).

ADAMTS-like protein 2 (ADAMTSL2) is a member of the ADAMTS (a disintegrin and metalloproteinase with thrombospondin motifs)-like protein subfamily and is a glycoprotein that binds to the cell surface and the extracellular matrix. ADAMTSL2 interacts with LTBP1 (latent transforming growth factor beta binding protein 1), and since the LTBP1 protein is involved in the storage of TGF-β1, which is an important growth factor that regulates the growth and division of cells, ADAMTSL2 regulates the availability of TGF-β1. In addition, ADAMTSL2 is expressed in cancer tissues and therefore used as a biomarker for cancer diagnosis.

In the present invention, the ADAMTSL2 may preferably include the amino acid sequence of SEQ ID NO: 1, or may include a sequence that is at least 90%, at least 93%, at least 95%, at least 96%, at least 97%, at least 98%, or at least 99% identical to the amino acid sequence of SEQ ID NO: 1.

Ceruloplasmin (Cp) plays an important role in iron metabolism as a major protein carrying copper in blood. At least about 95% of copper present in the plasma of healthy persons is in the form of ceruloplasmin.

In the present invention, the Cp may preferably include the amino acid sequence of SEQ ID NO: 2, or may include a sequence that is at least 90%, at least 93%, at least 95%, at least 96, at least 97%, at least 98%, or at least 99% identical to the amino acid sequence of SEQ ID NO: 3.

Complement factor H (CFH) is a glycoprotein that plays an essential role in regulating complement activation to maintain an immune response. It serves as a complement inhibitor that binds to the same sugar chain structure on the cell surface and prevents complement activation and amplification.

In the present invention, the CFH may preferably include the amino acid sequence of SEQ ID NO: 3, or may include a sequence that is at least 90%, at least 93%, at least 95%, at least 96, at least 97%, at least 98%, or at least 99% identical to the amino acid sequence of SEQ ID NO: 3.

Protein DDI1 homolog 2 (DDI2) is an internal peptide cleaving enzyme that activates nuclear respiratory factor 1 (Nrf1), which is involved in the regulation of cell growth and DNA replication, to complement protein degradation caused by abnormalities in the proteasome.

In the present invention, the DDI2 may preferably include the amino acid sequence of SEQ ID NO: 4, or may include a sequence that is at least 90%, at least 93%, at least 95%, at least 96%, at least 97%, at least 98%, or at least 99% identical to the amino acid sequence of SEQ ID NO: 4.

Ficolin 2 (FCN2) is a type of oligolectin and is composed of a short N-terminal part-collagen-like domain and a fibrinogen-like domain. It is mainly expressed in the liver and is known to play an important role in the lectin pathway of the complement system by binding to N-acetylglucosamine in the bacterial cell wall and acting as an opsonin like mannose-binding protein.

In the present invention, the FCN2 may preferably include the amino acid sequence of SEQ ID NO: 5, or may include a sequence that is at least 90%, at least 93%, at least 95%, at least 96%, at least 97%, at least 98%, or at least 99% identical to the amino acid sequence of SEQ ID NO: 5.

Insulin-like growth factor binding protein 2 (IGFBP2) binds to insulin-like growth factor (IGF) and modulates a variety of processes in the cell and thereby regulates angiogenesis. In addition, it is known to promote the growth of cancer cells in various cancers (prostate cancer, breast cancer, *etc*.).

In the present invention, the IGFBP2 may preferably include the amino acid sequence of SEQ ID NO: 6, or may include a sequence that is at least 90%, at least 93%, at least 95%, at least 96%, at least 97%, at least 98%, or at least 99% identical to the amino acid sequence of SEQ ID NO: 6.

Galectin-3-binding protein (LGALS3BP) is a protein encoded by the LGALS3BP gene and specifically binds to human macrophage-associated lectin (Mac-2) and galectin 1. LGALS3BP is known to be increased in the serum of cancer patients and HIV-infected patients and is involved in immune responses associated with the cytotoxicity of natural killer (NK) cells and lymphokine-activated killer (LAK) cells.

In the present invention, the LGALS3B may preferably include the amino acid sequence of SEQ ID NO: 7, or may include a sequence that is at least 90%, at least 93%, at least 95%, at least 96%, at least 97%, at least 98%, or at least 99% identical to the amino acid sequence of SEQ ID NO: 7.

Mannose-binding protein C (MBL2) is also referred to as mannose-binding lectin (MBL) or mannan-binding protein (MBP). MBL2 has an oligomeric structure (400 to 700 kDa) and is composed of subunits containing three identical peptide chains composed of approximately 30 kDa. It is produced by the liver in response to infection and is part of many other factors called acute-phase proteins.

In the present invention, the MBL2 may preferably include the amino acid sequence of SEQ ID NO: 8, or may include a sequence that is at least 90%, at least 93%, at least 95%, at least 96%, at least 97%, at least 98%, or at least 99% identical to the amino acid sequence of SEQ ID NO: 8.

Pancreatic triglyceride lipase (PNLIP) is a group of lipolytic enzymes that hydrolyze the ester bonds of triglycerides and is an enzyme secreted in the pancreas. Although PNLIP is known to be low in serum concentration because it is secreted into the duodenum through the pancreas duct system, it is known that when pancreatic functions are extremely disrupted by pancreatitis or pancreatic adenocarcinoma, pancreatic enzymes including PNLIP are secreted into serum, and thus, the PNLIP serum concentration may be measured to diagnose acute pancreatitis.

In the present invention, the PNLIP may preferably include the amino acid sequence of SEQ ID NO: 9, or may include a sequence that is at least 90%, at least 93%, at least 95%, at least 96%, at least 97%, at least 98%, or at least 99% identical to the amino acid sequence of SEQ ID NO:9.

E-selectin (SELE) is known as CD62 antigen-like family member E (CD62E), endothelial-leukocyte adhesion molecule 1 (ELAM) or leukocyte-endothelial cell adhesion molecule 2 (LECAM2), and it is a cell adhesion molecule that is transiently expressed and induced in vascular endothelial cells activated by interleukin 1β, tumor necrosis factor and lipopolysaccharide, peaking at 4 to 12 hours. SELE is strongly expressed in vascular endothelial cells in the inflamed tissue and mediates the rolling phenomenon of neutrophils and monocytes on vascular endothelial cells, thereby promoting infiltration of these cells into the inflammatory site. It is also known to be involved in the adhesion of cancer cells to vascular endothelial cells.

In the present invention, the SELE may preferably include the amino acid sequence of SEQ ID NO: 10, or may include a sequence that is at least 90%, at least 93%, at least 95%, at least 96%, at least 97%, at least 98%, or at least 99% identical to the amino acid sequence of SEQ ID NO: 10.

Sialic acid-binding Ig-like lectin 14 (SIGLEC14) is one of the subfamilies of sialic acid-binding immunoglobulin-type lectins (SIGLEC), and SIGLEC is a cell surface protein that binds to sialic acid and is mainly expressed on the surface of immune cells. The protein interaction between SIGLEC and sialic acid acts as a switch that turns the immune system on and off, and it is known that cancer cells also acquire resistance to the immune response using the SIGLEC-sialic acid reaction.

In the present invention, the SIGLEC14 may preferably include the amino acid sequence of SEQ ID NO: 11, or may include a sequence that is at least 90%, at least 93%, at least 95%, at least 96%, at least 97%, at least 98%, or at least 99% identical to the amino acid sequence of SEQ ID NO: 11.

Thrombospondin-1 (THBS1) is one of the thrombospondin families, and it is a glycoprotein that inhibits angiogenesis and tumorigenesis. It is known that it binds to proteases associated with angiogenesis such as plasminogen, urokinase, MMP, thrombin, cathepsin and the like, in order to regulate the adhesion, migration and growth of endothelial cells.

In the present invention, the THBS1 may preferably include the amino acid sequence of SEQ ID NO: 12, or may include a sequence that is at least 90%, at least 93%, at least 95%, at least 96%, at least 97%, at least 98%, or at least 99% identical to the amino acid sequence of SEQ ID NO: 12.

Zymogen granule protein 16 homolog B (ZG16B) is a pancreatic adenocarcinoma upregulated factor (PAUF), and it is known to bind to carbohydrates and activate the angiogenesis and permeability of endothelial cells.

In the present invention, the ZG16B may preferably include the amino acid sequence of SEQ ID NO: 13, or may include a sequence that is at least 90%, at least 93%, at least 95%, at least 96%, least 97%, at least 98%, or at least 99% identical to the amino acid sequence of SEQ ID NO: 13.

However, there is no known prior art that discloses that the above biomarkers may be used for the diagnosis of age-related macular degeneration, and in particular, there is no known technique that discloses that the combined biomarker of IGFBP2, ADAMTSL2, LGALS3BP and CFH may be used to increase the diagnostic performance of age-related macular degeneration.

In another aspect, the present invention relates to a composition for diagnosing age-related macular degeneration, including an agent for measuring the mRNA or protein level of a combined biomarker for diagnosing age-related macular degeneration, which includes insulin-like growth factor binding protein 2 (IGFBP2), ADAMTS-like protein 2 (ADAMTSL2), galectin 3 binding protein (LGALS3BP) and complement factor H (CFH).

In the present invention, the combined biomarker may be characterized by further including one or more biomarkers selected from the group consisting of ceruloplasmin (Cp), protein DDI1 homolog 2 (DDI2), ficolin 2 (FCN2), galectin 3 binding protein (LGALS3BP), mannose-binding protein C (MBL2), pancreatic triacylglycerol lipase (PNLIP), E-selectin (SELE), sialic acid-binding Ig-like lectin 14 (SIGLEC14), thrombospondin-1 (THBS1) and zymogen granule protein 16 homolog B (ZG16B).

In the present invention, the agent for measuring the mRNA level of the combined biomarker is characterized to be a primer pair, probe or antisense nucleotide that specifically binds to the genes of the biomarkers, and since the nucleic acid information of the genes is known in GeneBank and the like, a person skilled in the art may design such a primer pair, probe or antisense nucleotide based on the sequence.

As used herein, the term "measurement of mRNA expression level" is to measure the amount of mRNA in a biological sample isolated from a patient suspected of age-related macular degeneration in order to diagnose the age-related macular degeneration by determining the presence of mRNA and the degree of expression of genes for diagnosing age-related macular degeneration. The analysis method therefor may include reverse transcription polymerase chain reaction (RT-PCR), competitive reverse transcription polymerase chain reaction (competitive RT-PCR), real-time reverse transcription polymerase chain reaction (real-time RT-PCR), RNase protection assay (RPA), Northern blotting, DNA chip and the like, but is not limited thereto.

As used herein, the term "primer" is a fragment that recognizes a target gene sequence and includes forward and reverse primer pairs, and preferably, it is a primer pair that provides assay results with specificity and sensitivity. High specificity may be imparted when the nucleic acid sequence of the primer is a sequence that is mismatched with the non-target sequence present in the sample such that only the target gene sequence containing a complementary primer binding site is amplified and the primer does not cause non-specific amplification.

As used herein, the term "probe" means a substance capable of specifically binding to a target substance to be detected in a sample, and means a substance capable of specifically confirming the presence of the target substance in the sample through the binding. The type of probe is not limited as a material commonly used in the art, but may preferably be peptide nucleic acid (PNA), locked nucleic acid (LNA), peptide, polypeptide, protein, RNA or DNA, and most preferably PNA. More specifically, the probe includes those derived from or similar to an organism as a biomaterial or those prepared *ex vivo,* and may be, for example, an enzyme, protein, antibody, microorganism, animal and plant cells and organs, neuron, DNA, and RNA. In addition, DNA may include cDNA, genomic DNA and oligonucleotides, RNA may include genomic RNA, mRNA and oligonucleotide, and examples of proteins may include antibodies, antigens, enzymes, peptides and the like.

As used herein, the term "antisense" refers to an oligomer having a sequence of nucleotide bases and an inter-subunit backbone such that the antisense oligomer hybridizes to a target sequence within the RNA by the Watson-Crick base pairing, thereby allowing the formation of mRNA and RNA:oligomer heterodimers typically within the target sequence. The oligomer may have exact sequence complementarity or approximate complementarity to a target sequence.

In the present invention, the agent for measuring the protein level of the combined biomarker may be an antibody, interacting protein, ligand, nanoparticle or aptamer that specifically binds to the protein or peptide fragment.

As used herein, the term "measurement of protein expression level" is a process for determining the presence and the degree of expression of proteins expressed from genes for diagnosing age-related macular degeneration in a biological sample in order to diagnose age-related macular degeneration.

As used herein, the term "antibody" refers to a substance that specifically binds to an antigen and causes an antigen-antibody reaction. For the purposes of the present invention, an antibody means an antibody which binds specifically to the combined biomarker for diagnosing age-related macular degeneration according to the present invention. The antibodies of the present invention include all of polyclonal antibodies, monoclonal antibodies and recombinant antibodies. The antibodies may be easily prepared using techniques well known in the art. For example, the polyclonal antibodies may be produced by a method well known in the art, including the process of injecting the protein antigen of the biomarker for age-related macular degeneration into an animal and collecting blood from the animal to obtain a serum containing the antibody. Such polyclonal antibodies may be prepared from any animal such as a goat, rabbit, sheep, monkey, horse, pig, cow, dog and the like. In addition, monoclonal antibodies may be prepared using the hybridoma method well known in the art (refer to Kohler and Milstein, European Journal of Immunology 6:511-519, 1976) or the phage antibody library technique (refer to Clackson et al., Nature, 352:624-628, 1991; Marks et al., J. Mol. Biol., 222:58, 1-597, 1991). The antibody produced by the above method may be isolated and purified using methods such as gel electrophoresis, dialysis, salt precipitation, ion exchange chromatography, affinity chromatography and the like. In addition, the antibodies of the present invention include functional fragments of antibody molecules as well as complete forms having two full-length light chains and two full-length heavy chains. A functional fragment of an antibody molecule means a fragment having at least an antigen-binding function, and examples thereof include Fab, F(ab'), F(ab')2, Fv and the like. The antibodies of the present invention may also be commercially obtained.

As used herein, the term "peptide nucleic acid (PNA)" refers to an artificially synthesized DNA or RNA-like polymer. While DNA has a phosphate-ribose backbone, PNA has a repeated N-(2-aminoethyl)-glycine backbone linked by peptide bonds, which greatly increases the binding capacity and stability to DNA or RNA, and thus, it is used in molecular biology, diagnostic assays and antisense therapies. PNA is described in detail in the following document (Nielsen PE et al., Science, 254(5037): 1497-500, 1991).

In the present invention, an "aptamer" is an oligonucleotide or peptide molecule, and the general content of aptamers is described in detail in the following documents (Bock LC et al., Nature, 355(6360):5646, 1992; Hoppe-Seyler F and Butz K, J Mol Med., 78(8):42630, 2000; Cohen BA et al., Proc Natl Acad Sci USA., 95(24): 142727, 1998).

In another aspect, the present invention relates to a kit for diagnosing age-related macular degeneration, including the composition for diagnosing age-related macular degeneration.

The kit may be prepared by the conventional manufacturing methods known in the art. The kit may include, for example, an antibody in lyophilized form and a buffer, a stabilizer, an inert protein and the like.

The kit may further include a detectable label. The term "detectable label" refers to an atom or molecule that specifically detects a molecule including a label among the same type of molecules without the label. The detectable label may be one attached to an antibody, interacting protein, ligand, nanoparticle, or aptamer that specifically binds to the protein or fragment thereof. The detectable label may include a radionuclide, a fluorophore or an enzyme.

The kit may be used according to various immunoassays or immunostaining methods known in the art. The immunoassays or immunostaining may include radioimmunoassay, radioimmunoprecipitation, immunoprecipitation, ELISA, capture-ELISA, inhibition or competition assays, sandwich assays, flow cytometry, immunofluorescence and immunoaffinity purification. Preferably, the kit may be a reverse transcription polymerase chain reaction (RT-PCR) kit, a DNA chip kit, an enzyme-linked immunosorbent assay (ELISA) kit, a protein chip kit, a rapid kit or a multiple reaction monitoring (MRM) kit.

In addition, the kit may be used for mass spectrometry. In this case, certain amino acid residues of the protein may have modifications such as myristoylation, isoprenylation, prenylation, glypication, lipoylation, acylation, alkylation, methylation, demethylation, amidation, ubiquitination, phosphorylation, deamidation, glycosylation, oxidation, acetylation or the like.

In another aspect, the present invention relates to a method for providing information for diagnosing age-related macular degeneration, including the steps of (a) measuring the mRNA or protein level of a combined biomarker for diagnosing retinopathy comprising insulin-like growth factor binding protein 2 (IGFBP2), ADAMTS-like protein 2 (ADAMTSL2), galectin 3 binding protein (LGALS3BP) and complement factor H (CFH) from a biological sample of a patient; and (b) comparing the mRNA or protein expression level with an mRNA or protein expression level from a sample of a control group.

In the present invention, the combined biomarker of step (a) further includes one or more biomarkers selected from the group consisting of ceruloplasmin (Cp), protein DDI1 homolog 2 (DDI2), ficolin 2 (FCN2), galectin 3 binding protein (LGALS3BP), mannose-binding protein C (MBL2), pancreatic triacylglycerol lipase (PNLIP), E-selectin (SELE), sialic acid-binding Ig-like lectin 14 (SIGLEC14) and zymogen granule protein 16 homolog B (ZG16B).

In the above method, the term "biological sample" means a tissue, cell, blood, serum, plasma, saliva, cerebrospinal fluid, urine or the like, whose protein expression level or gene expression level differs by the onset of age-related macular degeneration, and it preferably means blood, plasma or serum.

The method for providing information for diagnosing age-related macular degeneration may further include one or more types of clinical information selected from the group consisting of the patient's age, body mass index (BMI), hypertension, hyperlipidemia, smoking status and cardiovascular disease.

In addition, the method for providing information for diagnosing age-related macular degeneration may further include the step of diagnosing age-related macular degeneration if the gene expression level or protein expression level of the combined biomarker increases compared to the control group.

The measurement of the mRNA expression level in step (a) may be measured and compared using a primer pair, a probe or an antisense nucleotide that specifically binds to the gene of the combined biomarker.

As the analysis method for measuring or comparing the mRNA expression level, the reverse transcription polymerase chain reaction, competitive reverse transcription polymerase chain reaction, real-time reverse transcription polymerase chain reaction, RNase protection assay, Northern blotting, DNA chip and the like may be used, but the present invention is not limited thereto. The above-described measurement methods may confirm the mRNA expression level of a normal control group and the mRNA expression level of age-related macular degeneration patients, and by comparing these expression levels, it is possible to diagnose or predict whether the onset of age-related macular degeneration has occurred.

The measurement of the protein expression level in step (a) may be measured and compared using antibodies, interacting proteins, ligands, nanoparticles or aptamers that specifically bind to a protein or peptide fragment.

Examples of the analysis method for measuring or comparing the protein expression level include the protein chip analysis, immunoassay, ligand binding assays, MALDI-TOF (matrix desorption/ionization time-of-flight mass spectrometry) analysis, SELDITOF (surface enhanced laser desorption or ionization time-of-fight mass spectrometry) analysis, radioimmunoassay, radioimmunodiffusion, Ouchterlony immunodiffusion, Rocket immunoelectrophoresis, tissue-immunostaining, complement fixation assay, two-dimensional electrophoresis analysis, liquid chromatography-mass spectrometry (LC-MS), liquid chromatography-mass spectrometry/mass spectrometry (LCMS/MS), Western blot, enzyme-linked immunosorbent assay (ELISA) and the like, but are not limited thereto

More preferably, in the present invention, it may be measured using multiple reaction monitoring (MRM), parallel reaction monitoring (PRM), sequential windowed data dependent acquisition of the total high-resolution (SWATH), selected reaction monitoring (SRM) or immune-multiple reaction monitoring (iMRM).

The MRM is a method of determining the exact fragment of a substance and breaking it in a mass spectrometer, and then selecting a specific ion from the broken ions once more to obtain the number using a continuously connected detector. When the MRM method is used, the corresponding protein or a fragment thereof may be quantified using a mass spectrometer in blood samples of a normal subject and a subject suspected of age-related macular degeneration.

The analysis of step (b) may be performed using a statistical method or algorithm to improve the accuracy of the diagnosis, and may use an analysis method selected from the group consisting of a linear or non-linear regression analysis method, a linear or non-linear classification analysis method, a logistic regression analysis method, an analysis of variance (ANOVA), a neural network analysis method, a genetic analysis method, a support vector machine analysis method, a hierarchical analysis or clustering analysis method, a hierarchical algorithm or kernel principal component analysis method using decision tree, the Markov Blanket analysis method, a recursive feature elimination or entropy-based regression feature elimination analysis method, a forward floating search or backward floating search analysis method, and combinations thereof. Preferably in the present invention, the statistical method used a logistic regression analysis method, but is not limited thereto.

In one specific embodiment of the present invention, 184 plasma samples were analyzed for quantitative validation of biomarkers for the diagnosis of age-related macular degeneration in plasma proteomes (Table 1), and 13 biomarkers consisting of insulin-like growth factor binding protein 2 (IGFBP2), ADAMTS-like protein 2 (ADAMTSL2), complement factor H (CFH), ceruloplasmin (Cp), protein DDI1 homolog 2 (DDI2), ficolin 2 (FCN2), galectin 3 binding protein (LGALS3BP), mannose-binding protein C (MBL2), pancreatic triacylglycerol lipase (PNLIP), E-selectin (SELE), sialic acid-binding Ig-like lectin 14 (SIGLEC14), thrombospodin-1 (THBS1) and zymogen granule protein 16 homolog B (ZG16B) were analyzed using multiple reaction monitoring (MRM). As shown in Table 2, MRM-MS analysis was performed using SIS by selecting peptides for each biomarker for quantitative analysis.

In another specific embodiment of the present invention, when the results of the combined biomarker group and the clinical information were combined, the probability value of being classified as age-related macular degeneration was estimated by inputting the conversion information of the biomarker expression level and the conversion values of the clinical information using a logistic regression model in order to confirm the improvement effect of diagnostic performance. As shown in Table 3, based on clinical information + IGFBP2 + ADAMTSL2 + LGALS3BP + CHF, the quantitative values for Cp, DDI2, FCN2, LGALS3BP, MBL2, SELE, SIGLEC14, PNLIP, THBS1 and ZG16B were added one by one and analyzed, and as a result, as the number of the biomarkers increased, it was confirmed that the diagnostic capacity of age-related macular degeneration increased.

In addition, as shown in FIG. 1 to FIG. 3 and Table 4, when the protein quantitative values for the above-mentioned 13 biomarkers and the basic clinical information were combined and statistically analyzed using the logistic regression model, it was confirmed that the diagnostic capacities for the normal group and age-related macular degeneration (STAGE 1, AUC=0.840), the normal group and early age-related macular degeneration (STAGE 2, AUC=0.810), and the normal group and late age-related macular degeneration (STAGE 3, AUC=0.859) were excellent.

In another specific embodiment of the invention, it was determined whether a difference in diagnostic performance was observed depending on the combinations of biomarkers. As shown in FIG. 2, it was confirmed that the combination of IGFBP2, ADAMTSL2, LGALS3BP and CFH (Combination 1, AUC=0.783) had higher diagnostic capacity than the combination of the 9 other proteins, Cp, DDI2, FCN2, MBL2, SIGLEC14, SELE, SIGLEC14, THBS1 and ZG16B (Combination 2, AUC=0.624) among the 13 targets.

Hereinafter, the present invention will be described in more detail through examples.

These examples are only for illustrating the present invention, and it will be apparent to those of ordinary skill in the art that the scope of the present invention is not to be construed as being limited by these examples.

### [Example 1]

### Selection and plasma collection of patients with age-related macular degeneration

Plasma samples from patients with age-related macular degeneration were obtained with approval by the Clinical Trial Review Committee of Seoul National University Bundang Hospital. A total of 184 plasma samples were analyzed for quantitative detection of biomarkers using plasma proteoms, and the clinical characteristics of the normal group (Non AMD) and the age-related macular degeneration (AMD) disease groups to be analyzed are shown in Table 1 below.

**[Table 1]**

| Clinical information of samples | | | | |
|---|---|---|---|---|
| | Sample (n=184) | | | |
| | Non AMD (n=32) | AMD (n=152) | Early AMD (n=57) | Late AMD (n=95) |
| Age | 59.3±8.1 | 71.2±8.3 | 72.1±6.7 | 70.7±9.1 |
| Gender (female/male) | 15/17 | 75/77 | 45/12 | 30/65 |
| Systemic risk factors | | | | |
| Smoking | 11 (44.4) | 61 (40.1) | 13 (22.8) | 48 (50.5) |
| Hypertension (%) | 10 (31.3) | 74 (48.7) | 23 (40.4) | 51 (53.7) |
| Hyperlipidemia (%) | 8 (25.0) | 54 (35.5) | 22 (38.6) | 32 (33.7) |
| Cardiovascular disease (%) | 3 (9.4) | 20 (13.2) | 8 (14.0) | 12 (12.6) |

### [Example 2]

### Peptide selection and peptide analysis using MRM-MS

### 2-1: Peptide selection and synthetic peptide design

In the present invention, as biomarkers for diagnosing age-related macular degeneration, 13 biomarkers of insulin-like growth factor binding protein 2 (IGFBP2), ADAMTS-like protein 2 (ADAMTSL2), complement factor H (CFH), ceruloplasmin (Cp), protein DDI1 homolog 2 (DDI2), ficolin 2 (FCN2), galectin 3 binding protein (LGALS3BP), mannose-binding protein C (MBL2), pancreatic triacylglycerol lipase (PNLIP), E-selectin (SELE), sialic acid-binding Ig-like lectin 14 (SIGLEC14), thrombospodin-1 (THBS1) and zymogen granule protein 16 homolog B (ZG16B) were selected.

In order to perform MRM analysis on the biomarkers, a representative peptide having a specific charge-to-mass ratio (m/z) for the proteins of the 13 biomarkers was selected (Q1), and among the fragmentation ions generated by breaking this peptide with an electric shock, the ion (Q3) with the highest strength was selected.

One or more peptides with high sensitivity per protein were measured and injected into a mass spectrometer based thereon to obtain the optimal fragmentation energy value for each transition, and three or more top fragmentation ions were selected based on the strength (Table 2).

**[Table 2]**

| Top fragmentation ions for 13 biomarkers | | | | |
|---|---|---|---|---|
| Name of gene | Trypsin fragment | SEQ ID NO. | Target ion | Target m/z |
| ADAMTSL2 | NFNIAGTVVK | SEQ ID NO: 14 | +2y6 | 531.801/574.356 |
| Cp | GEFYIGSK | SEQ ID NO: 15 | +2y6 | 450.728/714.382 |
| | AEVGDTI | SEQ ID NO: 16 | +2y5 | 430.727/561.299 |
| CFH | SLGNIIMVCR | SEQ ID NO: 17 | +2y5 | 581.807/678.343 |
| | SLGNVIMVCR | SEQ ID NO: 18 | +2y5 | 574.799/678.343 |
| | CYFPYLENGYNQNYGR | SEQ ID NO: 19 | +2y14+2 | 1029.444/867.897 |
| | CYFPYLENGYNQNHGR | SEQ ID NO: 20 | +3yl3+2 | 677.964/781.361 |
| DDI2 | DGDVVILR | SEQ ID NO: 21 | +2y4 | 443.753/500.355 |
| | IDFSSIAVPGTSSPR | SEQ ID NO: 22 | +2y7 | 767.399/701.358 |
| FCN2 | LQAADTCPEVK | SEQ ID NO: 23 | +2y8 | 616.303/919.419 |
| IGFBP2 | LIQGAPTIR | SEQ ID NO: 24 | +2y6 | 484.798/614.362 |
| LGALS3BP | SDLAVPSELALLK | SEQ ID NO: 25 | +2y8 | 678.393/870.529 |
| MBL2 | WLTFSLGK | SEQ ID NO: 26 | +2y6 | 476.269/652.366 |
| PNLIP | TGYTQASQNIR | SEQ ID NO: 27 | +2y6 | 619.81/688.374 |
| | GEENWLANVCK | SEQ ID NO: 28 | +2y5 | 660.306/591.292 |
| | VTGHILVSLFGNK | SEQ ID NO: 29 | +3y4 | 462.270/465.246 |
| SELE | NWAPGEPNNR | SEQ ID NO: 30 | +2y7 | 577.771/783.374 |
| | QPQNGSVR | SEQ ID NO: 31 | +2y6 | 443.230/660.342 |
| SIGLEC14 | EGGEFTCR | SEQ ID NO: 32 | +2y3 | 478.201/436.197 |
| THBS1 | GGVNDNFQGVLQNVR | SEQ ID NO: 33 | +2y7 | 808.911/785.463 |
| ZG16B | YFSTTEDYDHEITGLR | SEQ ID NO: 34 | +3y7 | 649.63/825.458 |

Stable-isotope labeled standard (SIS) peptides corresponding to 13 proteins were used to confirm quantification, and the spiked heavy peptide concentration was determined using an experiment to confirm linearity via calibration. The SIS peptide is a peptide in which 12C and 14N at the C-terminal lysine (Lys, K) or arginine (Arg, R) amino acid of the peptide are substituted with 13C and 15N. It differs in mass from endogenous peptides present in the blood, but since it has the same sequence, the peptide hydrophobicity is identical, and thus, it elutes at the same retention time (RT) on a chromatogram.

### 2-2: Pretreatment of plasma samples and MRM-MS analysis

Each plasma obtained in Example 1 above was used as it was, or for more accurate protein quantification, depletion was performed to remove 14 proteins (albumin, IgG, antitrypsin, IgA, transferrin, haptoglobin, fibrinogen, alpha 2-macroglobulin, alpha 1-acid glycoprotein, IgM, apolipoprotein AI, apolipoprotein All, complement C3 and transthyretin) present in high amounts. For depletion, the multiple affinity removal system (MARS, Agilent, USA) column was used according to the manufacturer's protocol to remove the 14 proteins and the remaining proteins were eluted in a small amount and used for analysis. Urea was added to the eluted depletion sample to 8 M, and then 2-carboxyethyltriphosphine (tris(2-carboxyethyl)-phosphine, TECP) was added to 80 mM and 2-chloroacetamide was added to 320 mM, respectively, and it was reacted at 25°C for 1 hour to reduce and alkylate disulfide bonds. LysC enzyme was added thereto such that the mass ratio of the plasma protein and LysC (wako) enzyme was 100:1, and it was reacted at room temperature for 4 hours. Afterwards, it was diluted 10-fold with a 50 mM Tris (pH 8.0) solution and trypsin (Promega) was added at a sequencing-able level such that the mass ratio of the protein and trypsin became 50:1, and it was reacted at 37°C for 12 hours or more and digested into peptide fragments. A formic acid solution was added to be 0.3% to acidify at pH of 2.0 or less, and the trypsin reaction was stopped.

Herein, desalination was carried out, and a heavy-labeled peptide was added (spiking) as an internal standard material (SIS peptide) determined in Example 2-1, and MRM analysis was performed. It was coupled to Nano ultra 2D plus (Eksigent) and was monitored in the scheduled MRM mode for the transition of each selected protein using the QTarp 5500 (SCIEX) equipment, which is a triple quadrupole mass spectrometer.

Specifically, an ion voltage of 5,500 volts was used, and the resolution at Quadruple 1 (Q1) and Quadruple 3 (Q3) was set in units. The transition was set such that the total cycle time was 1.5 seconds. 20 units of nebulizing gas were used and the heater temperature was set to 350°C. In order to correct for batch-to-batch variation, each sample was spiked with an internal standard material and monitored simultaneously.

### 2-3: Data analysis

Raw data was processed using Skyline (McCoss lab, University of Washington, USA) to calculate the peak area of the transition. Relative concentrations were compared using peak areas for the endogenous/heavy labeled peptide. In order to measure the predictive ability of each protein peptide on disease using the measured result values, T-test and AUROC (area under the receiver operating characteristic) values were generated, and in order to confirm the predictive ability combined with the results of the combined biomarker groups and the clinical information, the conversion information of the expression levels and the degree of conversion of the clinical information of Table 1 were inputted using a logistic regression model to estimate the probability values that were classified as age-related macular degeneration. All statistical analyses were performed using MedCal ver. 17.1 (MedCalc).

The clinical information reflected the body mass index (BMI) calculated by the height and body weight, smoking status and the presence of hyperlipidemia, hypertension and cardiovascular disease obtained through questionnaire. Among these, the information used for the presence or absence was converted to Yes = 1 and No = 0 (stop smoking = 0.5) and reflected.

### [Example 3]

### Confirmation of improvement in diagnostic capacity by combining results of combined biomarker groups and clinical information

In the present invention, in order to confirm the improvement effect of the diagnostic capacity when the results of the 13 combined biomarkers and the clinical information were combined, the probability value of being classified as age-related macular degeneration was estimated by inputting the conversion information of the biomarker expression levels and the degree of conversion of the clinical information using a logistic regression model.

**[Table 3]**

| Confirmation of diagnostic performance of age-related macular degeneration according to the combination number of combined biomarkers | | | | | | | | | | | | | | |
|---|---|---|---|---|---|---|---|---|---|---|---|---|---|---|
| C1 | | | | | C2 | C3 | C4 | C5 | C6 | C7 | C8 | C9 | C10 | AUC |
| 1 | 2 | 3 | 4 | 5 | | | | | | | | | | 0.778 |
| 1 | 2 | 3 | 4 | 5 | 6 | | | | | | | | | 0.779 |
| 1 | 2 | 3 | 4 | 5 | 6 | 7 | | | | | | | | 0.782 |
| 1 | 2 | 3 | 4 | 5 | 6 | 7 | 8 | | | | | | | 0.787 |
| 1 | 2 | 3 | 4 | 5 | 6 | 7 | 8 | 9 | | | | | | 0.788 |
| 1 | 2 | 3 | 4 | 5 | 6 | 7 | 8 | 9 | 10 | | | | | 0.821 |
| 1 | 2 | 3 | 4 | 5 | 6 | 7 | 8 | 9 | 10 | 11 | | | | 0.822 |
| 1 | 2 | 3 | 4 | 5 | 6 | 7 | 8 | 9 | 10 | 11 | 12 | | | 0.825 |
| 1 | 2 | 3 | 4 | 5 | 6 | 7 | 8 | 9 | 10 | 11 | 12 | 13 | | 0.828 |
| 1 | 2 | 3 | 4 | 5 | 6 | 7 | 8 | 9 | 10 | 11 | 12 | 13 | 14 | 0.840 |

| | | | | | | | | | | | | | | |
|---|---|---|---|---|---|---|---|---|---|---|---|---|---|---|
| 1. Clinical Information, 2. IGFBP2, 3. ADAMTSL2, 4. LGALS3BP, 5. CFH, 6. THBS, 7. SIGLEC14, 8. CP, 9. SELE, 10. DDI2, 11. MBL2, 12. PNLIP, 13. ZG16B, 14. FNC2 | | | | | | | | | | | | | | |

**[Table 4]**

| Confirmation of diagnostic capacity according to the combination of combined biomarker groups and clinical information | | | | | | | | | |
|---|---|---|---|---|---|---|---|---|---|
| | Non AMD vs AMD (STAGE 1) | | | Non AMD vs Early AMD (STAGE 2) | | | Non AMD vs Late AMD (STAGE 3) | | |
| | AUC | SE | 95% CI | AUC | SE | 95% CI | AUC | SE | 95% CI |
| C | 0.609 | 0.0529 | 3.506 to 0.713 | 0.546 | 0.0628 | 0.423 to 0.669 | 0.648 | 0.0557 | 0.538 to 0.757 |
| P | 0.827 | 0.0390 | 0.761 to 0.914 | 0.808 | 0.0475 | 0.714 to 0.901 | 0.855 | 0.0404 | 0.776 to 0.934 |
| Co m | 0.840 | 0.0385 | 0.765 to 0.916 | 0.810 | 0.0470 | 0.718 to J.902 | 0.859 | 0.0390 | 0.782 to J.935 |

| | | | | | | | | | |
|---|---|---|---|---|---|---|---|---|---|
| C: Clinical Information, P: Protein Quantitative Information, Com: Combination of Clinical Information and Protein Quantitative Information, AUC: Area Under the Curve, SE: Standard Error (Delong *et al.,* 1988), 95% CI: 95% Confidence Interval | | | | | | | | | |

As shown in Table 3, based on clinical information + IGFBP2 + LGALS3BP + ADAMTSL2 + CFH, the quantitative values for Cp, DDI2, FCN2, MBL2, SIGLEC14, SELE, SIGLEC14, THBS1 and ZG16B were added one by one and analyzed, and as a result, as the number of the biomarkers increased, it was confirmed that the diagnostic capacity (AUC) of age-related macular degeneration increased.

In addition, as shown in FIG. 1 to FIG. 3 and Table 4, when the protein quantitative values for the above-mentioned 13 biomarkers and the basic clinical information were combined and statistically processed using the logistic regression model, it was confirmed that the diagnostic capacity (AUC=0.840, FIG. 1) for the normal group versus age-related macular degeneration (Non AMD vs AMD), the diagnostic capacity (AUC=0.810, FIG. 2) of the normal group versus early age-related macular degeneration (Non AMD vs early AMD), and the diagnostic capacity (AUC=0.859) of the normal group versus late age-related macular degeneration (Non AMD vs late AMD) were all excellent.

### [Example 4]

### Confirmation of diagnostic performance of age-related macular degeneration according to the combination of biomarkers

In the present invention, it was confirmed whether there was a difference in the diagnostic performances of age-related macular degeneration according to the combinations of the 13 biomarkers. The 13 biomarkers were divided into a combination of IGFBP2, LGALS3BP, ADAMTSL2 and CFH and a combination of Cp, DDI2, FCN2, MBL2, SELE, SIGLEC14, THBS1 and ZG16B to perform analysis, and the analysis was performed together with clinical information in the same manner as in STAGE 1 of Example 3.

As shown in FIG. 4, it was confirmed that the combination of IGFBP2, LGALS3BP, ADAMTSL2 and CFH (Combination 1, AUC=0.778) had higher diagnostic capacity than the combination of the 9 other proteins, Cp, DDI2, FCN2, MBL2, SELE, SIGLEC14, THBS1 and ZG16B (Combination 2, AUC=0.624) among the 13 biomarkers.

### [Industrial Applicability]

The combined biomarker for diagnosing age-related macular degeneration according to the present invention was confirmed to exhibit excellent sensitivity and diagnostic performance compared to other biomarker combinations, and it was also confirmed to exhibit high diagnostic capacity in two stages of early and late age-related macular degeneration when the protein quantitative values of the combined biomarker and the basic clinical information were combined and analyzed. Thus, the present invention will be useful for early diagnosis of age-related macular degeneration.

## Claims

1. A combined biomarker for diagnosing age-related macular degeneration, comprising insulin-like growth factor binding protein 2 (IGFBP2), ADAMTS-like protein 2 (ADAMTSL2), galectin 3 binding protein (LGALS3BP) and complement factor H (CFH).

2. The combined biomarker of claim 1, wherein the combined biomarker further comprises one or more biomarkers selected from the group consisting of ceruloplasmin (Cp), protein DDI1 homolog 2 (DDI2), ficolin 2 (FCN2), mannose-binding protein C (MBL2), pancreatic triacylglycerol lipase (PNLIP), E-selectin (SELE), sialic acid-binding Ig-like lectin 14 (SIGLEC14), thrombospondin-1 (THBS1) and zymogen granule protein 16 homolog B (ZG16B).

3. A composition for diagnosing age-related macular degeneration, comprising an agent for measuring the mRNA or protein level of a combined biomarker for diagnosing age-related macular degeneration, the combined biomarker comprising insulin-like growth factor binding protein 2 (IGFBP2), ADAMTS-like protein 2 (ADAMTSL2), galectin 3 binding protein (LGALS3BP) and complement factor H (CFH).

4. The composition of claim 3, wherein the composition further comprises an agent for measuring the mRNA or protein level of one or more biomarkers selected from the group consisting of ceruloplasmin (Cp), protein DDI1 homolog 2 (DDI2), ficolin 2 (FCN2), mannose-binding protein C (MBL2), pancreatic triacylglycerol lipase (PNLIP), E-selectin (SELE), sialic acid-binding Ig-like lectin 14 (SIGLEC14), thrombospondin-1 (THBS1) and zymogen granule protein 16 homolog B (ZG16B).

5. The composition of claim 3, wherein the agent for measuring the mRNA level of the combined biomarker is a primer pair, probe or antisense nucleotide that specifically binds to a gene of each biomarker.

6. The composition of claim 3, wherein the agent for measuring the protein level of the combined biomarker is an antibody, interacting protein, ligand, nanoparticle or aptamer that specifically binds to a protein or peptide fragment of each biomarker.

7. A kit for diagnosing age-related macular degeneration, comprising the composition according to any one of claims 3 to 6.

8. The kit of claim 7, wherein the kit is a reverse transcription polymerase chain reaction (RT-PCR) kit, a DNA chip kit, an enzyme-linked immunosorbent assay (ELISA) kit, a protein chip kit, a rapid kit or a multiple reaction monitoring (MRM) kit.

9. A method for providing information for diagnosing age-related macular degeneration, comprising:
(a) measuring the mRNA or protein level of the combined biomarker of claim 1 or 2 from a biological sample of a patient; and
(b) comparing the mRNA or protein expression level with an mRNA or protein expression level from a sample of a control group.

10. The method of claim 9, wherein the method compares with the control group by further including one or more types of clinical information selected from the group consisting of the patient's age, body mass index (BMI), smoking status, hypertension, hyperlipidemia and cardiovascular disease.

11. The method of claim 9, wherein the method further comprises (c) diagnosing age-related macular degeneration if the gene expression level or protein expression level of the combined biomarker increases compared to the control group.


<110>    Reti Mark Co.

<120>    Combined Biomarkers for Diagnosing of Age Related Macular
         Degeneration and Use Thereof

<130>    1067160

<150>    KR 10-2019-0026303
<151>    2019-03-07

<160>    34

<170>    KoPatentIn 3.0

<210>    1
<211>    951
<212>    PRT
<213>    Artificial Sequence

<220>
<223>    ADAMTS-like protein 2


<400>    1


<210>    2
<211>    1065
<212>    PRT
<213>    Artificial Sequence

<220>
<223>    Ceruloplasmin


<400>    2
Met Lys Ile Leu Ile Leu Gly Ile Phe Leu Phe Leu Cys Ser Thr Pro
  1               5                  10                  15

Ala Trp Ala Lys Glu Lys His Tyr Tyr Ile Gly Ile Ile Glu Thr Thr
             20                  25                  30

Trp Asp Tyr Ala Ser Asp His Gly Glu Lys Lys Leu Ile Ser Val Asp
         35                  40                  45

Thr Glu His Ser Asn Ile Tyr Leu Gln Asn Gly Pro Asp Arg Ile Gly
     50                  55                  60

Arg Leu Tyr Lys Lys Ala Leu Tyr Leu Gln Tyr Thr Asp Glu Thr Phe
 65                  70                  75                  80

Arg Thr Thr Ile Glu Lys Pro Val Trp Leu Gly Phe Leu Gly Pro Ile
                 85                  90                  95

Ile Lys Ala Glu Thr Gly Asp Lys Val Tyr Val His Leu Lys Asn Leu
            100                 105                 110

Ala Ser Arg Pro Tyr Thr Phe His Ser His Gly Ile Thr Tyr Tyr Lys
        115                 120                 125

Glu His Glu Gly Ala Ile Tyr Pro Asp Asn Thr Thr Asp Phe Gln Arg
    130                 135                 140

Ala Asp Asp Lys Val Tyr Pro Gly Glu Gln Tyr Thr Tyr Met Leu Leu
145                 150                 155                 160

Ala Thr Glu Glu Gln Ser Pro Gly Glu Gly Asp Gly Asn Cys Val Thr
                165                 170                 175

Arg Ile Tyr His Ser His Ile Asp Ala Pro Lys Asp Ile Ala Ser Gly
            180                 185                 190

Leu Ile Gly Pro Leu Ile Ile Cys Lys Lys Asp Ser Leu Asp Lys Glu

195                 200                 205

Lys Glu Lys His Ile Asp Arg Glu Phe Val Val Met Phe Ser Val Val
    210                 215                 220

Asp Glu Asn Phe Ser Trp Tyr Leu Glu Asp Asn Ile Lys Thr Tyr Cys
225                 230                 235                 240

Ser Glu Pro Glu Lys Val Asp Lys Asp Asn Glu Asp Phe Gln Glu Ser
                245                 250                 255

Asn Arg Met Tyr Ser Val Asn Gly Tyr Thr Phe Gly Ser Leu Pro Gly
            260                 265                 270

Leu Ser Met Cys Ala Glu Asp Arg Val Lys Trp Tyr Leu Phe Gly Met
        275                 280                 285

Gly Asn Glu Val Asp Val His Ala Ala Phe Phe His Gly Gln Ala Leu
    290                 295                 300

Thr Asn Lys Asn Tyr Arg Ile Asp Thr Ile Asn Leu Phe Pro Ala Thr
305                 310                 315                 320

Leu Phe Asp Ala Tyr Met Val Ala Gln Asn Pro Gly Glu Trp Met Leu
                325                 330                 335

Ser Cys Gln Asn Leu Asn His Leu Lys Ala Gly Leu Gln Ala Phe Phe
            340                 345                 350

Gln Val Gln Glu Cys Asn Lys Ser Ser Ser Lys Asp Asn Ile Arg Gly
        355                 360                 365

Lys His Val Arg His Tyr Tyr Ile Ala Ala Glu Glu Ile Ile Trp Asn
    370                 375                 380

Tyr Ala Pro Ser Gly Ile Asp Ile Phe Thr Lys Glu Asn Leu Thr Ala
385                 390                 395                 400

Pro Gly Ser Asp Ser Ala Val Phe Phe Glu Gln Gly Thr Thr Arg Ile
                405                 410                 415

Gly Gly Ser Tyr Lys Lys Leu Val Tyr Arg Glu Tyr Thr Asp Ala Ser
            420                 425                 430

Phe Thr Asn Arg Lys Glu Arg Gly Pro Glu Glu Glu His Leu Gly Ile
        435                 440                 445

Leu Gly Pro Val Ile Trp Ala Glu Val Gly Asp Thr Ile Arg Val Thr
    450                 455                 460

Phe His Asn Lys Gly Ala Tyr Pro Leu Ser Ile Glu Pro Ile Gly Val
465                 470                 475                 480

Arg Phe Asn Lys Asn Asn Glu Gly Thr Tyr Tyr Ser Pro Asn Tyr Asn
                485                 490                 495

Pro Gln Ser Arg Ser Val Pro Pro Ser Ala Ser His Val Ala Pro Thr
            500                 505                 510

Glu Thr Phe Thr Tyr Glu Trp Thr Val Pro Lys Glu Val Gly Pro Thr
        515                 520                 525

Asn Ala Asp Pro Val Cys Leu Ala Lys Met Tyr Tyr Ser Ala Val Asp

530                 535                 540

Pro Thr Lys Asp Ile Phe Thr Gly Leu Ile Gly Pro Met Lys Ile Cys
545                 550                 555                 560

Lys Lys Gly Ser Leu His Ala Asn Gly Arg Gln Lys Asp Val Asp Lys
                565                 570                 575

Glu Phe Tyr Leu Phe Pro Thr Val Phe Asp Glu Asn Glu Ser Leu Leu
            580                 585                 590

Leu Glu Asp Asn Ile Arg Met Phe Thr Thr Ala Pro Asp Gln Val Asp
        595                 600                 605

Lys Glu Asp Glu Asp Phe Gln Glu Ser Asn Lys Met His Ser Met Asn
    610                 615                 620

Gly Phe Met Tyr Gly Asn Gln Pro Gly Leu Thr Met Cys Lys Gly Asp
625                 630                 635                 640

Ser Val Val Trp Tyr Leu Phe Ser Ala Gly Asn Glu Ala Asp Val His
                645                 650                 655

Gly Ile Tyr Phe Ser Gly Asn Thr Tyr Leu Trp Arg Gly Glu Arg Arg
            660                 665                 670

Asp Thr Ala Asn Leu Phe Pro Gln Thr Ser Leu Thr Leu His Met Trp
        675                 680                 685

Pro Asp Thr Glu Gly Thr Phe Asn Val Glu Cys Leu Thr Thr Asp His
    690                 695                 700

Tyr Thr Gly Gly Met Lys Gln Lys Tyr Thr Val Asn Gln Cys Arg Arg
705                 710                 715                 720

Gln Ser Glu Asp Ser Thr Phe Tyr Leu Gly Glu Arg Thr Tyr Tyr Ile
                725                 730                 735

Ala Ala Val Glu Val Glu Trp Asp Tyr Ser Pro Gln Arg Glu Trp Glu
            740                 745                 750

Lys Glu Leu His His Leu Gln Glu Gln Asn Val Ser Asn Ala Phe Leu
        755                 760                 765

Asp Lys Gly Glu Phe Tyr Ile Gly Ser Lys Tyr Lys Lys Val Val Tyr
    770                 775                 780

Arg Gln Tyr Thr Asp Ser Thr Phe Arg Val Pro Val Glu Arg Lys Ala
785                 790                 795                 800

Glu Glu Glu His Leu Gly Ile Leu Gly Pro Gln Leu His Ala Asp Val
                805                 810                 815

Gly Asp Lys Val Lys Ile Ile Phe Lys Asn Met Ala Thr Arg Pro Tyr
            820                 825                 830

Ser Ile His Ala His Gly Val Gln Thr Glu Ser Ser Thr Val Thr Pro
        835                 840                 845

Thr Leu Pro Gly Glu Thr Leu Thr Tyr Val Trp Lys Ile Pro Glu Arg
    850                 855                 860

Ser Gly Ala Gly Thr Glu Asp Ser Ala Cys Ile Pro Trp Ala Tyr Tyr

865                 870                 875                 880

Ser Thr Val Asp Gln Val Lys Asp Leu Tyr Ser Gly Leu Ile Gly Pro
                885                 890                 895

Leu Ile Val Cys Arg Arg Pro Tyr Leu Lys Val Phe Asn Pro Arg Arg
            900                 905                 910

Lys Leu Glu Phe Ala Leu Leu Phe Leu Val Phe Asp Glu Asn Glu Ser
        915                 920                 925

Trp Tyr Leu Asp Asp Asn Ile Lys Thr Tyr Ser Asp His Pro Glu Lys
    930                 935                 940

Val Asn Lys Asp Asp Glu Glu Phe Ile Glu Ser Asn Lys Met His Ala
945                 950                 955                 960

Ile Asn Gly Arg Met Phe Gly Asn Leu Gln Gly Leu Thr Met His Val
                965                 970                 975

Gly Asp Glu Val Asn Trp Tyr Leu Met Gly Met Gly Asn Glu Ile Asp
            980                 985                 990

Leu His Thr Val His Phe His Gly His Ser Phe Gln Tyr Lys His Arg
        995                 1000                1005

Gly Val Tyr Ser Ser Asp Val Phe Asp Ile Phe Pro Gly Thr Tyr Gln
    1010                1015                1020

Thr Leu Glu Met Phe Pro Arg Thr Pro Gly Ile Trp Leu Leu His Cys
1025                1030                1035                1040

His Val Thr Asp His Ile His Ala Gly Met Glu Thr Thr Tyr Thr Val
                1045                1050                1055

Leu Gln Asn Glu Asp Thr Lys Ser Gly
            1060                1065


<210>   3
<211>   1231
<212>   PRT
<213>   Artificial Sequence

<220>
<223>   Complement factor H sequence


<400>   3
Met Arg Leu Leu Ala Lys Ile Ile Cys Leu Met Leu Trp Ala Ile Cys
1               5                   10                  15

Val Ala Glu Asp Cys Asn Glu Leu Pro Pro Arg Arg Asn Thr Glu Ile
            20                  25                  30

Leu Thr Gly Ser Trp Ser Asp Gln Thr Tyr Pro Glu Gly Thr Gln Ala
        35                  40                  45

Ile Tyr Lys Cys Arg Pro Gly Tyr Arg Ser Leu Gly Asn Val Ile Met
    50                  55                  60

Val Cys Arg Lys Gly Glu Trp Val Ala Leu Asn Pro Leu Arg Lys Cys
65                  70                  75                  80

Gln Lys Arg Pro Cys Gly His Pro Gly Asp Thr Pro Phe Gly Thr Phe
                85                  90                  95

Thr Leu Thr Gly Gly Asn Val Phe Glu Tyr Gly Val Lys Ala Val Tyr
            100                 105                 110

Thr Cys Asn Glu Gly Tyr Gln Leu Leu Gly Glu Ile Asn Tyr Arg Glu
        115                 120                 125

Cys Asp Thr Asp Gly Trp Thr Asn Asp Ile Pro Ile Cys Glu Val Val
    130                 135                 140

Lys Cys Leu Pro Val Thr Ala Pro Glu Asn Gly Lys Ile Val Ser Ser
145                 150                 155                 160

Ala Met Glu Pro Asp Arg Glu Tyr His Phe Gly Gln Ala Val Arg Phe
                165                 170                 175

Val Cys Asn Ser Gly Tyr Lys Ile Glu Gly Asp Glu Glu Met His Cys
            180                 185                 190

Ser Asp Asp Gly Phe Trp Ser Lys Glu Lys Pro Lys Cys Val Glu Ile
        195                 200                 205

Ser Cys Lys Ser Pro Asp Val Ile Asn Gly Ser Pro Ile Ser Gln Lys
    210                 215                 220

Ile Ile Tyr Lys Glu Asn Glu Arg Phe Gln Tyr Lys Cys Asn Met Gly
225                 230                 235                 240

Tyr Glu Tyr Ser Glu Arg Gly Asp Ala Val Cys Thr Glu Ser Gly Trp
                245                 250                 255

Arg Pro Leu Pro Ser Cys Glu Glu Lys Ser Cys Asp Asn Pro Tyr Ile
            260                 265                 270

Pro Asn Gly Asp Tyr Ser Pro Leu Arg Ile Lys His Arg Thr Gly Asp
        275                 280                 285

Glu Ile Thr Tyr Gln Cys Arg Asn Gly Phe Tyr Pro Ala Thr Arg Gly
    290                 295                 300

Asn Thr Ala Lys Cys Thr Ser Thr Gly Trp Ile Pro Ala Pro Arg Cys
305                 310                 315                 320

Thr Leu Lys Pro Cys Asp Tyr Pro Asp Ile Lys His Gly Gly Leu Tyr
                325                 330                 335

His Glu Asn Met Arg Arg Pro Tyr Phe Pro Val Ala Val Gly Lys Tyr
            340                 345                 350

Tyr Ser Tyr Tyr Cys Asp Glu His Phe Glu Thr Pro Ser Gly Ser Tyr
        355                 360                 365

Trp Asp His Ile His Cys Thr Gln Asp Gly Trp Ser Pro Ala Val Pro
    370                 375                 380

Cys Leu Arg Lys Cys Tyr Phe Pro Tyr Leu Glu Asn Gly Tyr Asn Gln
385                 390                 395                 400

Asn Tyr Gly Arg Lys Phe Val Gln Gly Lys Ser Ile Asp Val Ala Cys
                405                 410                 415

His Pro Gly Tyr Ala Leu Pro Lys Ala Gln Thr Thr Val Thr Cys Met
        420                 425                 430

Glu Asn Gly Trp Ser Pro Thr Pro Arg Cys Ile Arg Val Lys Thr Cys
    435                 440                 445

Ser Lys Ser Ser Ile Asp Ile Glu Asn Gly Phe Ile Ser Glu Ser Gln
    450                 455                 460

Tyr Thr Tyr Ala Leu Lys Glu Lys Ala Lys Tyr Gln Cys Lys Leu Gly
465                 470                 475                 480

Tyr Val Thr Ala Asp Gly Glu Thr Ser Gly Ser Ile Thr Cys Gly Lys
                485                 490                 495

Asp Gly Trp Ser Ala Gln Pro Thr Cys Ile Lys Ser Cys Asp Ile Pro
            500                 505                 510

Val Phe Met Asn Ala Arg Thr Lys Asn Asp Phe Thr Trp Phe Lys Leu
        515                 520                 525

Asn Asp Thr Leu Asp Tyr Glu Cys His Asp Gly Tyr Glu Ser Asn Thr
    530                 535                 540

Gly Ser Thr Thr Gly Ser Ile Val Cys Gly Tyr Asn Gly Trp Ser Asp
545                 550                 555                 560

Leu Pro Ile Cys Tyr Glu Arg Glu Cys Glu Leu Pro Lys Ile Asp Val
                565                 570                 575

His Leu Val Pro Asp Arg Lys Lys Asp Gln Tyr Lys Val Gly Glu Val
            580                 585                 590

Leu Lys Phe Ser Cys Lys Pro Gly Phe Thr Ile Val Gly Pro Asn Ser
        595                 600                 605

Val Gln Cys Tyr His Phe Gly Leu Ser Pro Asp Leu Pro Ile Cys Lys
    610                 615                 620

Glu Gln Val Gln Ser Cys Gly Pro Pro Pro Glu Leu Leu Asn Gly Asn
625                 630                 635                 640

Val Lys Glu Lys Thr Lys Glu Glu Tyr Gly His Ser Glu Val Val Glu
                645                 650                 655

Tyr Tyr Cys Asn Pro Arg Phe Leu Met Lys Gly Pro Asn Lys Ile Gln
            660                 665                 670

Cys Val Asp Gly Glu Trp Thr Thr Leu Pro Val Cys Ile Val Glu Glu
        675                 680                 685

Ser Thr Cys Gly Asp Ile Pro Glu Leu Glu His Gly Trp Ala Gln Leu
    690                 695                 700

Ser Ser Pro Pro Tyr Tyr Tyr Gly Asp Ser Val Glu Phe Asn Cys Ser
705                 710                 715                 720

Glu Ser Phe Thr Met Ile Gly His Arg Ser Ile Thr Cys Ile His Gly
                725                 730                 735

Val Trp Thr Gln Leu Pro Gln Cys Val Ala Ile Asp Lys Leu Lys Lys
            740                 745                 750

Cys Lys Ser Ser Asn Leu Ile Ile Leu Glu Glu His Leu Lys Asn Lys
        755                 760                 765

Lys Glu Phe Asp His Asn Ser Asn Ile Arg Tyr Arg Cys Arg Gly Lys
    770                 775                 780

Glu Gly Trp Ile His Thr Val Cys Ile Asn Gly Arg Trp Asp Pro Glu
785                 790                 795                 800

Val Asn Cys Ser Met Ala Gln Ile Gln Leu Cys Pro Pro Pro Pro Gln
                805                 810                 815

Ile Pro Asn Ser His Asn Met Thr Thr Thr Leu Asn Tyr Arg Asp Gly
            820                 825                 830

Glu Lys Val Ser Val Leu Cys Gln Glu Asn Tyr Leu Ile Gln Glu Gly
        835                 840                 845

Glu Glu Ile Thr Cys Lys Asp Gly Arg Trp Gln Ser Ile Pro Leu Cys
    850                 855                 860

Val Glu Lys Ile Pro Cys Ser Gln Pro Pro Gln Ile Glu His Gly Thr
865                 870                 875                 880

Ile Asn Ser Ser Arg Ser Ser Gln Glu Ser Tyr Ala His Gly Thr Lys
                885                 890                 895

Leu Ser Tyr Thr Cys Glu Gly Gly Phe Arg Ile Ser Glu Glu Asn Glu
            900                 905                 910

Thr Thr Cys Tyr Met Gly Lys Trp Ser Ser Pro Pro Gln Cys Glu Gly
        915                 920                 925

Leu Pro Cys Lys Ser Pro Pro Glu Ile Ser His Gly Val Val Ala His
    930                 935                 940

Met Ser Asp Ser Tyr Gln Tyr Gly Glu Glu Val Thr Tyr Lys Cys Phe
945                 950                 955                 960

Glu Gly Phe Gly Ile Asp Gly Pro Ala Ile Ala Lys Cys Leu Gly Glu
                965                 970                 975

Lys Trp Ser His Pro Pro Ser Cys Ile Lys Thr Asp Cys Leu Ser Leu
            980                 985                 990

Pro Ser Phe Glu Asn Ala Ile Pro Met Gly Glu Lys Lys Asp Val Tyr
        995                 1000                1005

Lys Ala Gly Glu Gln Val Thr Tyr Thr Cys Ala Thr Tyr Tyr Lys Met
    1010                1015                1020

Asp Gly Ala Ser Asn Val Thr Cys Ile Asn Ser Arg Trp Thr Gly Arg
1025                1030                1035                1040

Pro Thr Cys Arg Asp Thr Ser Cys Val Asn Pro Pro Thr Val Gln Asn
                1045                1050                1055

Ala Tyr Ile Val Ser Arg Gln Met Ser Lys Tyr Pro Ser Gly Glu Arg
            1060                1065                1070

Val Arg Tyr Gln Cys Arg Ser Pro Tyr Glu Met Phe Gly Asp Glu Glu
        1075                1080                1085

Val Met Cys Leu Asn Gly Asn Trp Thr Glu Pro Pro Gln Cys Lys Asp
    1090                1095                1100

Ser Thr Gly Lys Cys Gly Pro Pro Pro Pro Ile Asp Asn Gly Asp Ile
1105                1110                1115                1120

Thr Ser Phe Pro Leu Ser Val Tyr Ala Pro Ala Ser Ser Val Glu Tyr
                1125                1130                1135

Gln Cys Gln Asn Leu Tyr Gln Leu Glu Gly Asn Lys Arg Ile Thr Cys
            1140                1145                1150

Arg Asn Gly Gln Trp Ser Glu Pro Pro Lys Cys Leu His Pro Cys Val
        1155                1160                1165

Ile Ser Arg Glu Ile Met Glu Asn Tyr Asn Ile Ala Leu Arg Trp Thr
    1170                1175                1180

Ala Lys Gln Lys Leu Tyr Ser Arg Thr Gly Glu Ser Val Glu Phe Val
1185                1190                1195                1200

Cys Lys Arg Gly Tyr Arg Leu Ser Ser Arg Ser His Thr Leu Arg Thr
                1205                1210                1215

Thr Cys Trp Asp Gly Lys Leu Glu Tyr Pro Thr Cys Ala Lys Arg
            1220                1225                1230

<210>    4
<211>    399
<212>    PRT
<213>    Artificial Sequence

<220>
<223>    Protein DDI1 homolog2

<400>    4
Met Leu Leu Thr Val Tyr Cys Val Arg Arg Asp Leu Ser Glu Val Thr
1               5                   10                  15

Phe Ser Leu Gln Val Asp Ala Asp Phe Glu Leu His Asn Phe Arg Ala
            20                  25                  30

Leu Cys Glu Leu Glu Ser Gly Ile Pro Ala Ala Glu Ser Gln Ile Val
        35                  40                  45

Tyr Ala Glu Arg Pro Leu Thr Asp Asn His Arg Ser Leu Ala Ser Tyr
    50                  55                  60

Gly Leu Lys Asp Gly Asp Val Val Ile Leu Arg Gln Lys Glu Asn Ala
65                  70                  75                  80

Asp Pro Arg Pro Pro Val Gln Phe Pro Asn Leu Pro Arg Ile Asp Phe
                85                  90                  95

Ser Ser Ile Ala Val Pro Gly Thr Ser Ser Pro Arg Gln Arg Gln Pro
            100                 105                 110

Pro Gly Thr Gln Gln Ser His Ser Ser Pro Gly Glu Ile Thr Ser Ser
        115                 120                 125

Pro Gln Gly Leu Asp Asn Pro Ala Leu Leu Arg Asp Met Leu Leu Ala
    130                 135                 140

Asn Pro His Glu Leu Ser Leu Leu Lys Glu Arg Asn Pro Pro Leu Ala
145                 150                 155                 160

Glu Ala Leu Leu Ser Gly Asp Leu Glu Lys Phe Ser Arg Val Leu Val
                165                 170                 175

Glu Gln Gln Gln Asp Arg Ala Arg Arg Glu Gln Glu Arg Ile Arg Leu
            180                 185                 190

Phe Ser Ala Asp Pro Phe Asp Leu Glu Ala Gln Ala Lys Ile Glu Glu
        195                 200                 205

Asp Ile Arg Gln Gln Asn Ile Glu Glu Asn Met Thr Ile Ala Met Glu
    210                 215                 220

Glu Ala Pro Glu Ser Phe Gly Gln Val Val Met Leu Tyr Ile Asn Cys
225                 230                 235                 240

Lys Val Asn Gly His Pro Val Lys Ala Phe Val Asp Ser Gly Ala Gln
                245                 250                 255

Met Thr Ile Met Ser Gln Ala Cys Ala Glu Arg Cys Asn Ile Met Arg
            260                 265                 270

Leu Val Asp Arg Arg Trp Ala Gly Ile Ala Lys Gly Val Gly Thr Gln
        275                 280                 285

Lys Ile Ile Gly Arg Val His Leu Ala Gln Val Gln Ile Glu Gly Asp
    290                 295                 300

Phe Leu Pro Cys Ser Phe Ser Ile Leu Glu Glu Gln Pro Met Asp Met
305                 310                 315                 320

Leu Leu Gly Leu Asp Met Leu Lys Arg His Gln Cys Ser Ile Asp Leu
                325                 330                 335

Lys Lys Asn Val Leu Val Ile Gly Thr Thr Gly Ser Gln Thr Thr Phe
            340                 345                 350

Leu Pro Glu Gly Glu Leu Pro Glu Cys Ala Arg Leu Ala Tyr Gly Ala
        355                 360                 365

Gly Arg Glu Asp Val Arg Pro Glu Glu Ile Ala Asp Gln Glu Leu Ala
    370                 375                 380

Glu Ala Leu Gln Lys Ser Ala Glu Asp Ala Glu Arg Gln Lys Pro
385                 390                 395


<210>    5
<211>    313
<212>    PRT
<213>    Artificial Sequence

<220>
<223>    Ficolin 2


<400>    5

Met Glu Leu Asp Arg Ala Val Gly Val Leu Gly Ala Ala Thr Leu Leu
1               5                   10                  15

Leu Ser Phe Leu Gly Met Ala Trp Ala Leu Gln Ala Ala Asp Thr Cys
            20                  25                  30

Pro Glu Val Lys Met Val Gly Leu Glu Gly Ser Asp Lys Leu Thr Ile
        35                  40                  45

Leu Arg Gly Cys Pro Gly Leu Pro Gly Ala Pro Gly Pro Lys Gly Glu
    50                  55                  60

Ala Gly Thr Asn Gly Lys Arg Gly Glu Arg Gly Pro Pro Gly Pro Pro
65                  70                  75                  80

Gly Lys Ala Gly Pro Pro Gly Pro Asn Gly Ala Pro Gly Glu Pro Gln
                85                  90                  95

Pro Cys Leu Thr Gly Pro Arg Thr Cys Lys Asp Leu Leu Asp Arg Gly
            100                 105                 110

His Phe Leu Ser Gly Trp His Thr Ile Tyr Leu Pro Asp Cys Arg Pro
        115                 120                 125

Leu Thr Val Leu Cys Asp Met Asp Thr Asp Gly Gly Gly Trp Thr Val
    130                 135                 140

Phe Gln Arg Arg Val Asp Gly Ser Val Asp Phe Tyr Arg Asp Trp Ala
145                 150                 155                 160

Thr Tyr Lys Gln Gly Phe Gly Ser Arg Leu Gly Glu Phe Trp Leu Gly
                165                 170                 175

Asn Asp Asn Ile His Ala Leu Thr Ala Gln Gly Thr Ser Glu Leu Arg
            180                 185                 190

Val Asp Leu Val Asp Phe Glu Asp Asn Tyr Gln Phe Ala Lys Tyr Arg
        195                 200                 205

Ser Phe Lys Val Ala Asp Glu Ala Glu Lys Tyr Asn Leu Val Leu Gly
    210                 215                 220

Ala Phe Val Glu Gly Ser Ala Gly Asp Ser Leu Thr Phe His Asn Asn
225                 230                 235                 240

Gln Ser Phe Ser Thr Lys Asp Gln Asp Asn Asp Leu Asn Thr Gly Asn
                245                 250                 255

Cys Ala Val Met Phe Gln Gly Ala Trp Trp Tyr Lys Asn Cys His Val
            260                 265                 270

Ser Asn Leu Asn Gly Arg Tyr Leu Arg Gly Thr His Gly Ser Phe Ala
        275                 280                 285

Asn Gly Ile Asn Trp Lys Ser Gly Lys Gly Tyr Asn Tyr Ser Tyr Lys
    290                 295                 300

Val Ser Glu Met Lys Val Arg Pro Ala
305                 310

<210>   6
<211>   325

<212>    PRT
<213>    Artificial Sequence

<220>
<223>    insulin like growth factor binding protein 2


<400>    6
Met Leu Pro Arg Val Gly Cys Pro Ala Leu Pro Leu Pro Pro Pro Pro
1               5                   10                  15

Leu Leu Pro Leu Leu Leu Leu Leu Leu Gly Ala Ser Gly Gly Gly Gly
            20                  25                  30

Gly Ala Arg Ala Glu Val Leu Phe Arg Cys Pro Pro Cys Thr Pro Glu
        35                  40                  45

Arg Leu Ala Ala Cys Gly Pro Pro Pro Val Ala Pro Pro Ala Ala Val
    50                  55                  60

Ala Ala Val Ala Gly Gly Ala Arg Met Pro Cys Ala Glu Leu Val Arg
65                  70                  75                  80

Glu Pro Gly Cys Gly Cys Cys Ser Val Cys Ala Arg Leu Glu Gly Glu
                85                  90                  95

Ala Cys Gly Val Tyr Thr Pro Arg Cys Gly Gln Gly Leu Arg Cys Tyr
            100                 105                 110

Pro His Pro Gly Ser Glu Leu Pro Leu Gln Ala Leu Val Met Gly Glu
        115                 120                 125

Gly Thr Cys Glu Lys Arg Arg Asp Ala Glu Tyr Gly Ala Ser Pro Glu
    130                 135                 140

Gln Val Ala Asp Asn Gly Asp Asp His Ser Glu Gly Gly Leu Val Glu
145                 150                 155                 160

Asn His Val Asp Ser Thr Met Asn Met Leu Gly Gly Gly Gly Ser Ala
                165                 170                 175

Gly Arg Lys Pro Leu Lys Ser Gly Met Lys Glu Leu Ala Val Phe Arg
            180                 185                 190

Glu Lys Val Thr Glu Gln His Arg Gln Met Gly Lys Gly Gly Lys His
        195                 200                 205

His Leu Gly Leu Glu Glu Pro Lys Lys Leu Arg Pro Pro Pro Ala Arg
    210                 215                 220

Thr Pro Cys Gln Gln Glu Leu Asp Gln Val Leu Glu Arg Ile Ser Thr
225                 230                 235                 240

Met Arg Leu Pro Asp Glu Arg Gly Pro Leu Glu His Leu Tyr Ser Leu
                245                 250                 255

His Ile Pro Asn Cys Asp Lys His Gly Leu Tyr Asn Leu Lys Gln Cys
            260                 265                 270

Lys Met Ser Leu Asn Gly Gln Arg Gly Glu Cys Trp Cys Val Asn Pro
        275                 280                 285

Asn Thr Gly Lys Leu Ile Gln Gly Ala Pro Thr Ile Arg Gly Asp Pro

290                 295                 300

Glu Cys His Leu Phe Tyr Asn Glu Gln Gln Glu Ala Arg Gly Val His
305                 310                 315                 320

Thr Gln Arg Met Gln
                325

<210>   7
<211>   585
<212>   PRT
<213>   Artificial Sequence

<220>
<223>   galectin-3-binding protein

<400>   7
Met Thr Pro Pro Arg Leu Phe Trp Val Trp Leu Leu Val Ala Gly Thr
1               5                   10                  15

Gln Gly Val Asn Asp Gly Asp Met Arg Leu Ala Asp Gly Gly Ala Thr
            20                  25                  30

Asn Gln Gly Arg Val Glu Ile Phe Tyr Arg Gly Gln Trp Gly Thr Val
        35                  40                  45

Cys Asp Asn Leu Trp Asp Leu Thr Asp Ala Ser Val Val Cys Arg Ala
    50                  55                  60

Leu Gly Phe Glu Asn Ala Thr Gln Ala Leu Gly Arg Ala Ala Phe Gly
65                  70                  75                  80

Gln Gly Ser Gly Pro Ile Met Leu Asp Glu Val Gln Cys Thr Gly Thr
                85                  90                  95

Glu Ala Ser Leu Ala Asp Cys Lys Ser Leu Gly Trp Leu Lys Ser Asn
            100                 105                 110

Cys Arg His Glu Arg Asp Ala Gly Val Val Cys Thr Asn Glu Thr Arg
        115                 120                 125

Ser Thr His Thr Leu Asp Leu Ser Arg Glu Leu Ser Glu Ala Leu Gly
    130                 135                 140

Gln Ile Phe Asp Ser Gln Arg Gly Cys Asp Leu Ser Ile Ser Val Asn
145                 150                 155                 160

Val Gln Gly Glu Asp Ala Leu Gly Phe Cys Gly His Thr Val Ile Leu
                165                 170                 175

Thr Ala Asn Leu Glu Ala Gln Ala Leu Trp Lys Glu Pro Gly Ser Asn
            180                 185                 190

Val Thr Met Ser Val Asp Ala Glu Cys Val Pro Met Val Arg Asp Leu
        195                 200                 205

Leu Arg Tyr Phe Tyr Ser Arg Arg Ile Asp Ile Thr Leu Ser Ser Val
    210                 215                 220

Lys Cys Phe His Lys Leu Ala Ser Ala Tyr Gly Ala Arg Gln Leu Gln
225                 230                 235                 240

Gly Tyr Cys Ala Ser Leu Phe Ala Ile Leu Leu Pro Gln Asp Pro Ser
                245                 250                 255

Phe Gln Met Pro Leu Asp Leu Tyr Ala Tyr Ala Val Ala Thr Gly Asp
            260                 265                 270

Ala Leu Leu Glu Lys Leu Cys Leu Gln Phe Leu Ala Trp Asn Phe Glu
        275                 280                 285

Ala Leu Thr Gln Ala Glu Ala Trp Pro Ser Val Pro Thr Asp Leu Leu
    290                 295                 300

Gln Leu Leu Leu Pro Arg Ser Asp Leu Ala Val Pro Ser Glu Leu Ala
305                 310                 315                 320

Leu Leu Lys Ala Val Asp Thr Trp Ser Trp Gly Glu Arg Ala Ser His
                325                 330                 335

Glu Glu Val Glu Gly Leu Val Glu Lys Ile Arg Phe Pro Met Met Leu
            340                 345                 350

Pro Glu Glu Leu Phe Glu Leu Gln Phe Asn Leu Ser Leu Tyr Trp Ser
        355                 360                 365

His Glu Ala Leu Phe Gln Lys Lys Thr Leu Gln Ala Leu Glu Phe His
    370                 375                 380

Thr Val Pro Phe Gln Leu Leu Ala Arg Tyr Lys Gly Leu Asn Leu Thr
385                 390                 395                 400

Glu Asp Thr Tyr Lys Pro Arg Ile Tyr Thr Ser Pro Thr Trp Ser Ala
                405                 410                 415

Phe Val Thr Asp Ser Ser Trp Ser Ala Arg Lys Ser Gln Leu Val Tyr
            420                 425                 430

Gln Ser Arg Arg Gly Pro Leu Val Lys Tyr Ser Ser Asp Tyr Phe Gln
        435                 440                 445

Ala Pro Ser Asp Tyr Arg Tyr Tyr Pro Tyr Gln Ser Phe Gln Thr Pro
    450                 455                 460

Gln His Pro Ser Phe Leu Phe Gln Asp Lys Arg Val Ser Trp Ser Leu
465                 470                 475                 480

Val Tyr Leu Pro Thr Ile Gln Ser Cys Trp Asn Tyr Gly Phe Ser Cys
                485                 490                 495

Ser Ser Asp Glu Leu Pro Val Leu Gly Leu Thr Lys Ser Gly Gly Ser
            500                 505                 510

Asp Arg Thr Ile Ala Tyr Glu Asn Lys Ala Leu Met Leu Cys Glu Gly
        515                 520                 525

Leu Phe Val Ala Asp Val Thr Asp Phe Glu Gly Trp Lys Ala Ala Ile
    530                 535                 540

Pro Ser Ala Leu Asp Thr Asn Ser Ser Lys Ser Thr Ser Ser Phe Pro
545                 550                 555                 560

Cys Pro Ala Gly His Phe Asn Gly Phe Arg Thr Val Ile Arg Pro Phe
                565                 570                 575

Tyr Leu Thr Asn Ser Ser Gly Val Asp
            580                 585

<210> 8
<211> 248
<212> PRT
<213> Artificial Sequence

<220>
<223> mannose-binding protein C

<400> 8
Met Ser Leu Phe Pro Ser Leu Pro Leu Leu Leu Leu Ser Met Val Ala
1               5                   10                  15

Ala Ser Tyr Ser Glu Thr Val Thr Cys Glu Asp Ala Gln Lys Thr Cys
            20                  25                  30

Pro Ala Val Ile Ala Cys Ser Ser Pro Gly Ile Asn Gly Phe Pro Gly
        35                  40                  45

Lys Asp Gly Arg Asp Gly Thr Lys Gly Glu Lys Gly Glu Pro Gly Gln
    50                  55                  60

Gly Leu Arg Gly Leu Gln Gly Pro Pro Gly Lys Leu Gly Pro Pro Gly
65                  70                  75                  80

Asn Pro Gly Pro Ser Gly Ser Pro Gly Pro Lys Gly Gln Lys Gly Asp
                85                  90                  95

Pro Gly Lys Ser Pro Asp Gly Asp Ser Ser Leu Ala Ala Ser Glu Arg
            100                 105                 110

Lys Ala Leu Gln Thr Glu Met Ala Arg Ile Lys Lys Trp Leu Thr Phe
        115                 120                 125

Ser Leu Gly Lys Gln Val Gly Asn Lys Phe Phe Leu Thr Asn Gly Glu
    130                 135                 140

Ile Met Thr Phe Glu Lys Val Lys Ala Leu Cys Val Lys Phe Gln Ala
145                 150                 155                 160

Ser Val Ala Thr Pro Arg Asn Ala Ala Glu Asn Gly Ala Ile Gln Asn
                165                 170                 175

Leu Ile Lys Glu Glu Ala Phe Leu Gly Ile Thr Asp Glu Lys Thr Glu
            180                 185                 190

Gly Gln Phe Val Asp Leu Thr Gly Asn Arg Leu Thr Tyr Thr Asn Trp
        195                 200                 205

Asn Glu Gly Glu Pro Asn Asn Ala Gly Ser Asp Glu Asp Cys Val Leu
    210                 215                 220

Leu Leu Lys Asn Gly Gln Trp Asn Asp Val Pro Cys Ser Thr Ser His
225                 230                 235                 240

Leu Ala Val Cys Glu Phe Pro Ile
                245

<210>    9
<211>    465
<212>    PRT
<213>    Artificial Sequence

<220>
<223>    pancreatic triacylglycerol lipase


<400>    9
Met Leu Pro Leu Trp Thr Leu Ser Leu Leu Leu Gly Ala Val Ala Gly
1               5                   10                  15

Lys Glu Val Cys Tyr Glu Arg Leu Gly Cys Phe Ser Asp Asp Ser Pro
            20                  25                  30

Trp Ser Gly Ile Thr Glu Arg Pro Leu His Ile Leu Pro Trp Ser Pro
        35                  40                  45

Lys Asp Val Asn Thr Arg Phe Leu Leu Tyr Thr Asn Glu Asn Pro Asn
    50                  55                  60

Asn Phe Gln Glu Val Ala Ala Asp Ser Ser Ser Ile Ser Gly Ser Asn
65                  70                  75                  80

Phe Lys Thr Asn Arg Lys Thr Arg Phe Ile Ile His Gly Phe Ile Asp
                85                  90                  95

Lys Gly Glu Glu Asn Trp Leu Ala Asn Val Cys Lys Asn Leu Phe Lys
            100                 105                 110

Val Glu Ser Val Asn Cys Ile Cys Val Asp Trp Lys Gly Gly Ser Arg
        115                 120                 125

Thr Gly Tyr Thr Gln Ala Ser Gln Asn Ile Arg Ile Val Gly Ala Glu
    130                 135                 140

Val Ala Tyr Phe Val Glu Phe Leu Gln Ser Ala Phe Gly Tyr Ser Pro
145                 150                 155                 160

Ser Asn Val His Val Ile Gly His Ser Leu Gly Ala His Ala Ala Gly
                165                 170                 175

Glu Ala Gly Arg Arg Thr Asn Gly Thr Ile Gly Arg Ile Thr Gly Leu
            180                 185                 190

Asp Pro Ala Glu Pro Cys Phe Gln Gly Thr Pro Glu Leu Val Arg Leu
        195                 200                 205

Asp Pro Ser Asp Ala Lys Phe Val Asp Val Ile His Thr Asp Gly Ala
    210                 215                 220

Pro Ile Val Pro Asn Leu Gly Phe Gly Met Ser Gln Val Val Gly His
225                 230                 235                 240

Leu Asp Phe Phe Pro Asn Gly Gly Val Glu Met Pro Gly Cys Lys Lys
                245                 250                 255

Asn Ile Leu Ser Gln Ile Val Asp Ile Asp Gly Ile Trp Glu Gly Thr
            260                 265                 270

Arg Asp Phe Ala Ala Cys Asn His Leu Arg Ser Tyr Lys Tyr Tyr Thr

275                 280                 285

Asp Ser Ile Val Asn Pro Asp Gly Phe Ala Gly Phe Pro Cys Ala Ser
    290                 295                 300

Tyr Asn Val Phe Thr Ala Asn Lys Cys Phe Pro Cys Pro Ser Gly Gly
305                 310                 315                 320

Cys Pro Gln Met Gly His Tyr Ala Asp Arg Tyr Pro Gly Lys Thr Asn
                325                 330                 335

Asp Val Gly Gln Lys Phe Tyr Leu Asp Thr Gly Asp Ala Ser Asn Phe
            340                 345                 350

Ala Arg Trp Arg Tyr Lys Val Ser Val Thr Leu Ser Gly Lys Lys Val
        355                 360                 365

Thr Gly His Ile Leu Val Ser Leu Phe Gly Asn Lys Gly Asn Ser Lys
    370                 375                 380

Gln Tyr Glu Ile Phe Lys Gly Thr Leu Lys Pro Asp Ser Thr His Ser
385                 390                 395                 400

Asn Glu Phe Asp Ser Asp Val Asp Val Gly Asp Leu Gln Met Val Lys
                405                 410                 415

Phe Ile Trp Tyr Asn Asn Val Ile Asn Pro Thr Leu Pro Arg Val Gly
            420                 425                 430

Ala Ser Lys Ile Ile Val Glu Thr Asn Val Gly Lys Gln Phe Asn Phe
        435                 440                 445

Cys Ser Pro Glu Thr Val Arg Glu Glu Val Leu Leu Thr Leu Thr Pro
    450                 455                 460

Cys
465


<210>   10
<211>   610
<212>   PRT
<213>   Artificial Sequence

<220>
<223>   E-selectin


<400>   10
Met Ile Ala Ser Gln Phe Leu Ser Ala Leu Thr Leu Val Leu Leu Ile
1               5                   10                  15

Lys Glu Ser Gly Ala Trp Ser Tyr Asn Thr Ser Thr Glu Ala Met Thr
            20                  25                  30

Tyr Asp Glu Ala Ser Ala Tyr Cys Gln Gln Arg Tyr Thr His Leu Val
        35                  40                  45

Ala Ile Gln Asn Lys Glu Glu Ile Glu Tyr Leu Asn Ser Ile Leu Ser
    50                  55                  60

Tyr Ser Pro Ser Tyr Tyr Trp Ile Gly Ile Arg Lys Val Asn Asn Val
65                  70                  75                  80

Trp Val Trp Val Gly Thr Gln Lys Pro Leu Thr Glu Glu Ala Lys Asn
                85                  90                  95

Trp Ala Pro Gly Glu Pro Asn Asn Arg Gln Lys Asp Glu Asp Cys Val
            100                 105                 110

Glu Ile Tyr Ile Lys Arg Glu Lys Asp Val Gly Met Trp Asn Asp Glu
        115                 120                 125

Arg Cys Ser Lys Lys Lys Leu Ala Leu Cys Tyr Thr Ala Ala Cys Thr
    130                 135                 140

Asn Thr Ser Cys Ser Gly His Gly Glu Cys Val Glu Thr Ile Asn Asn
145                 150                 155                 160

Tyr Thr Cys Lys Cys Asp Pro Gly Phe Ser Gly Leu Lys Cys Glu Gln
                165                 170                 175

Ile Val Asn Cys Thr Ala Leu Glu Ser Pro Glu His Gly Ser Leu Val
            180                 185                 190

Cys Ser His Pro Leu Gly Asn Phe Ser Tyr Asn Ser Ser Cys Ser Ile
        195                 200                 205

Ser Cys Asp Arg Gly Tyr Leu Pro Ser Ser Met Glu Thr Met Gln Cys
    210                 215                 220

Met Ser Ser Gly Glu Trp Ser Ala Pro Ile Pro Ala Cys Asn Val Val
225                 230                 235                 240

Glu Cys Asp Ala Val Thr Asn Pro Ala Asn Gly Phe Val Glu Cys Phe
                245                 250                 255

Gln Asn Pro Gly Ser Phe Pro Trp Asn Thr Thr Cys Thr Phe Asp Cys
            260                 265                 270

Glu Glu Gly Phe Glu Leu Met Gly Ala Gln Ser Leu Gln Cys Thr Ser
        275                 280                 285

Ser Gly Asn Trp Asp Asn Glu Lys Pro Thr Cys Lys Ala Val Thr Cys
    290                 295                 300

Arg Ala Val Arg Gln Pro Gln Asn Gly Ser Val Arg Cys Ser His Ser
305                 310                 315                 320

Pro Ala Gly Glu Phe Thr Phe Lys Ser Ser Cys Asn Phe Thr Cys Glu
                325                 330                 335

Glu Gly Phe Met Leu Gln Gly Pro Ala Gln Val Glu Cys Thr Thr Gln
            340                 345                 350

Gly Gln Trp Thr Gln Gln Ile Pro Val Cys Glu Ala Phe Gln Cys Thr
        355                 360                 365

Ala Leu Ser Asn Pro Glu Arg Gly Tyr Met Asn Cys Leu Pro Ser Ala
    370                 375                 380

Ser Gly Ser Phe Arg Tyr Gly Ser Ser Cys Glu Phe Ser Cys Glu Gln
385                 390                 395                 400

Gly Phe Val Leu Lys Gly Ser Lys Arg Leu Gln Cys Gly Pro Thr Gly
                405                 410                 415

Glu Trp Asp Asn Glu Lys Pro Thr Cys Glu Ala Val Arg Cys Asp Ala
            420                 425                 430

Val His Gln Pro Pro Lys Gly Leu Val Arg Cys Ala His Ser Pro Ile
        435                 440                 445

Gly Glu Phe Thr Tyr Lys Ser Ser Cys Ala Phe Ser Cys Glu Glu Gly
    450                 455                 460

Phe Glu Leu His Gly Ser Thr Gln Leu Glu Cys Thr Ser Gln Gly Gln
465                 470                 475                 480

Trp Thr Glu Glu Val Pro Ser Cys Gln Val Val Lys Cys Ser Ser Leu
                485                 490                 495

Ala Val Pro Gly Lys Ile Asn Met Ser Cys Ser Gly Glu Pro Val Phe
            500                 505                 510

Gly Thr Val Cys Lys Phe Ala Cys Pro Glu Gly Trp Thr Leu Asn Gly
        515                 520                 525

Ser Ala Ala Arg Thr Cys Gly Ala Thr Gly His Trp Ser Gly Leu Leu
    530                 535                 540

Pro Thr Cys Glu Ala Pro Thr Glu Ser Asn Ile Pro Leu Val Ala Gly
545                 550                 555                 560

Leu Ser Ala Ala Gly Leu Ser Leu Leu Thr Leu Ala Pro Phe Leu Leu
                565                 570                 575

Trp Leu Arg Lys Cys Leu Arg Lys Ala Lys Lys Phe Val Pro Ala Ser
            580                 585                 590

Ser Cys Gln Ser Leu Glu Ser Asp Gly Ser Tyr Gln Lys Pro Ser Tyr
        595                 600                 605

Ile Leu
    610


<210>    11
<211>    396
<212>    PRT
<213>    Artificial Sequence

<220>
<223>    Sialic acid-binding Ig-like lectin 14


<400>    11
Met Leu Pro Leu Leu Leu Leu Pro Leu Leu Trp Gly Gly Ser Leu Gln
1               5                   10                  15

Glu Lys Pro Val Tyr Glu Leu Gln Val Gln Lys Ser Val Thr Val Gln
            20                  25                  30

Glu Gly Leu Cys Val Leu Val Pro Cys Ser Phe Ser Tyr Pro Trp Arg
        35                  40                  45

Ser Trp Tyr Ser Ser Pro Pro Leu Tyr Val Tyr Trp Phe Arg Asp Gly
    50                  55                  60

Glu Ile Pro Tyr Tyr Ala Glu Val Val Ala Thr Asn Asn Pro Asp Arg
65                  70                  75                  80

Arg Val Lys Pro Glu Thr Gln Gly Arg Phe Arg Leu Leu Gly Asp Val
                85                  90                  95

Gln Lys Lys Asn Cys Ser Leu Ser Ile Gly Asp Ala Arg Met Glu Asp
            100                 105                 110

Thr Gly Ser Tyr Phe Phe Arg Val Glu Arg Gly Arg Asp Val Lys Tyr
        115                 120                 125

Ser Tyr Gln Gln Asn Lys Leu Asn Leu Glu Val Thr Ala Leu Ile Glu
    130                 135                 140

Lys Pro Asp Ile His Phe Leu Glu Pro Leu Glu Ser Gly Arg Pro Thr
145                 150                 155                 160

Arg Leu Ser Cys Ser Leu Pro Gly Ser Cys Glu Ala Gly Pro Pro Leu
                165                 170                 175

Thr Phe Ser Trp Thr Gly Asn Ala Leu Ser Pro Leu Asp Pro Glu Thr
            180                 185                 190

Thr Arg Ser Ser Glu Leu Thr Leu Thr Pro Arg Pro Glu Asp His Gly
        195                 200                 205

Thr Asn Leu Thr Cys Gln Val Lys Arg Gln Gly Ala Gln Val Thr Thr
    210                 215                 220

Glu Arg Thr Val Gln Leu Asn Val Ser Tyr Ala Pro Gln Asn Leu Ala
225                 230                 235                 240

Ile Ser Ile Phe Phe Arg Asn Gly Thr Gly Thr Ala Leu Arg Ile Leu
                245                 250                 255

Ser Asn Gly Met Ser Val Pro Ile Gln Glu Gly Gln Ser Leu Phe Leu
            260                 265                 270

Ala Cys Thr Val Asp Ser Asn Pro Pro Ala Ser Leu Ser Trp Phe Arg
        275                 280                 285

Glu Gly Lys Ala Leu Asn Pro Ser Gln Thr Ser Met Ser Gly Thr Leu
    290                 295                 300

Glu Leu Pro Asn Ile Gly Ala Arg Glu Gly Gly Glu Phe Thr Cys Arg
305                 310                 315                 320

Val Gln His Pro Leu Gly Ser Gln His Leu Ser Phe Ile Leu Ser Val
                325                 330                 335

Gln Arg Ser Ser Ser Ser Cys Ile Cys Val Thr Glu Lys Gln Gln Gly
            340                 345                 350

Ser Trp Pro Leu Val Leu Thr Leu Ile Arg Gly Ala Leu Met Gly Ala
        355                 360                 365

Gly Phe Leu Leu Thr Tyr Gly Leu Thr Trp Ile Tyr Tyr Thr Arg Cys
    370                 375                 380

Gly Gly Pro Gln Gln Ser Arg Ala Glu Arg Pro Gly
385                 390                 395

<210>    12
<211>    1170
<212>    PRT
<213>    Artificial Sequence

<220>
<223>    Thrombospondin-1


<400>    12



<210>    13
<211>    208
<212>    PRT
<213>    Artificial Sequence

<220>
<223>    Zymogen granule protein 16 homolog B


<400>    13
Met Gly Ala Gln Gly Ala Gln Glu Ser Ile Lys Ala Met Trp Arg Val
1               5                   10                  15

Pro Gly Thr Thr Arg Arg Pro Val Thr Gly Glu Ser Pro Gly Met His
            20                  25                  30

Arg Pro Glu Ala Met Leu Leu Leu Leu Thr Leu Ala Leu Leu Gly Gly
        35                  40                  45

Pro Thr Trp Ala Gly Lys Met Tyr Gly Pro Gly Gly Gly Lys Tyr Phe
    50                  55                  60

Ser Thr Thr Glu Asp Tyr Asp His Glu Ile Thr Gly Leu Arg Val Ser
65                  70                  75                  80

Val Gly Leu Leu Leu Val Lys Ser Val Gln Val Lys Leu Gly Asp Ser
                85                  90                  95

Trp Asp Val Lys Leu Gly Ala Leu Gly Gly Asn Thr Gln Glu Val Thr
            100                 105                 110

Leu Gln Pro Gly Glu Tyr Ile Thr Lys Val Phe Val Ala Phe Gln Ala
        115                 120                 125

Phe Leu Arg Gly Met Val Met Tyr Thr Ser Lys Asp Arg Tyr Phe Tyr
    130                 135                 140

Phe Gly Lys Leu Asp Gly Gln Ile Ser Ser Ala Tyr Pro Ser Gln Glu
145                 150                 155                 160

Gly Gln Val Leu Val Gly Ile Tyr Gly Gln Tyr Gln Leu Leu Gly Ile
                165                 170                 175

Lys Ser Ile Gly Phe Glu Trp Asn Tyr Pro Leu Glu Glu Pro Thr Thr
            180                 185                 190

Glu Pro Pro Val Asn Leu Thr Tyr Ser Ala Asn Ser Pro Val Gly Arg
        195                 200                 205

<210> 14
<211> 10
<212> PRT
<213> Artificial Sequence

<220>
<223> ADAMTSL2 peptide

<400> 14
Asn Phe Asn Ile Ala Gly Thr Val Val Lys
1               5                   10

<210> 15
<211> 8
<212> PRT
<213> Artificial Sequence

<220>
<223> Cp peptide

<400> 15
Gly Glu Phe Tyr Ile Gly Ser Lys
1               5

<210> 16

<211>    7
<212>    PRT
<213>    Artificial Sequence

<220>
<223>    Cp peptide


<400>    16
Ala Glu Val Gly Asp Thr Ile
1               5


<210>    17
<211>    10
<212>    PRT
<213>    Artificial Sequence

<220>
<223>    CFH peptide


<400>    17
Ser Leu Gly Asn Ile Ile Met Val Cys Arg
1               5                   10


<210>    18
<211>    10
<212>    PRT
<213>    Artificial Sequence

<220>
<223>    CFH peptide


<400>    18
Ser Leu Gly Asn Val Ile Met Val Cys Arg
1               5                   10


<210>    19
<211>    16
<212>    PRT
<213>    Artificial Sequence

<220>
<223>    CFH peptide


<400>    19
Cys Tyr Phe Pro Tyr Leu Glu Asn Gly Tyr Asn Gln Asn Tyr Gly Arg
1               5                   10                  15



<210>    20
<211>    16
<212>    PRT
<213>    Artificial Sequence

<220>
<223>    CFH peptide

<400>    20
Cys Tyr Phe Pro Tyr Leu Glu Asn Gly Tyr Asn Gln Asn His Gly Arg
1               5                   10                  15

<210>    21
<211>    8
<212>    PRT
<213>    Artificial Sequence

<220>
<223>    DDI2 peptide

<400>    21
Asp Gly Asp Val Val Ile Leu Arg
1               5

<210>    22
<211>    15
<212>    PRT
<213>    Artificial Sequence

<220>
<223>    DDI2 peptide

<400>    22
Ile Asp Phe Ser Ser Ile Ala Val Pro Gly Thr Ser Ser Pro Arg
1               5                   10                  15

<210>    23
<211>    11
<212>    PRT
<213>    Artificial Sequence

<220>
<223>    FCN2 peptide

<400>    23
Leu Gln Ala Ala Asp Thr Cys Pro Glu Val Lys
1               5                   10

<210>    24
<211>    9
<212>    PRT
<213>    Artificial Sequence

<220>
<223>    IGFBP2 peptide

<400>    24
Leu Ile Gln Gly Ala Pro Thr Ile Arg
1               5

<210>    25
<211>    13
<212>    PRT
<213>    Artificial Sequence

<220>
<223>    LGALS3BP peptide


<400>    25
Ser Asp Leu Ala Val Pro Ser Glu Leu Ala Leu Leu Lys
1               5                   10


<210>    26
<211>    8
<212>    PRT
<213>    Artificial Sequence

<220>
<223>    MBL2 peptide


<400>    26
Trp Leu Thr Phe Ser Leu Gly Lys
1               5


<210>    27
<211>    11
<212>    PRT
<213>    Artificial Sequence

<220>
<223>    PNLIP peptide


<400>    27
Thr Gly Tyr Thr Gln Ala Ser Gln Asn Ile Arg
1               5                   10


<210>    28
<211>    11
<212>    PRT
<213>    Artificial Sequence

<220>
<223>    PNLIP peptide


<400>    28
Gly Glu Glu Asn Trp Leu Ala Asn Val Cys Lys
1               5                   10


<210>    29
<211>    13
<212>    PRT
<213>    Artificial Sequence

<220>

<223>    PNLIP peptide

<400>    29
Val Thr Gly His Ile Leu Val Ser Leu Phe Gly Asn Lys
1               5                   10

<210>    30
<211>    10
<212>    PRT
<213>    Artificial Sequence

<220>
<223>    SELE peptide

<400>    30
Asn Trp Ala Pro Gly Glu Pro Asn Asn Arg
1               5                   10

<210>    31
<211>    8
<212>    PRT
<213>    Artificial Sequence

<220>
<223>    SELE peptide

<400>    31
Gln Pro Gln Asn Gly Ser Val Arg
1               5

<210>    32
<211>    8
<212>    PRT
<213>    Artificial Sequence

<220>
<223>    SIGLEC14 peptide

<400>    32
Glu Gly Gly Glu Phe Thr Cys Arg
1               5

<210>    33
<211>    15
<212>    PRT
<213>    Artificial Sequence

<220>
<223>    THBS1 peptide

<400>    33
Gly Gly Val Asn Asp Asn Phe Gln Gly Val Leu Gln Asn Val Arg
1               5                   10                  15

<210>    34
<211>    16
<212>    PRT
<213>    Artificial Sequence

<220>
<223>    ZG16B peptide

<400>    34
Tyr Phe Ser Thr Thr Glu Asp Tyr Asp His Glu Ile Thr Gly Leu Arg
1               5                   10                  15